(19) Europäisches Patentamt
European Patent Office
Office européen des brevets

(11) Veröffentlichungsnummer: **0 144 013**
**B1**

(12) # EUROPÄISCHE PATENTSCHRIFT

(45) Veröffentlichungstag der Patentschrift: **18.01.89**

(51) Int. Cl.⁴: **C 07 D 495/04**, C 07 D 519/00, C 09 K 19/34 // (C07D495/04, 333:00, 333:00)

(21) Anmeldenummer: **84113622.9**

(22) Anmeldetag: **12.11.84**

(54) **Thienothiophenderivate.**

(30) Priorität: **25.11.83 DE 3342631**

(43) Veröffentlichungstag der Anmeldung: **12.06.85 Patentblatt 85/24**

(45) Bekanntmachung des Hinweises auf die Patenterteilung: **18.01.89 Patentblatt 89/3**

(84) Benannte Vertragsstaaten: **AT CH DE FR GB IT LI NL**

(56) Entgegenhaltungen: **MOLECULAR CRYSTALS AND LIQUID CRYSTALS, Band 67, Nr. 1-4, 1981, Seiten 241-252, Gordon and Breach Science Publishers Inc., US; LA. KARAMYSHEVA u.a.: "New heterocyclic liquid crystalline compounds"**

(73) Patentinhaber: **Merck Patent Gesellschaft mit beschränkter Haftung, Frankfurter Strasse 250, D-6100 Darmstadt (DE)**

(72) Erfinder: **Krause, Joachim, Dr., Samuel-Morse-Strasse 14, D-6110 Dieburg (DE)**
Erfinder: **Römer, Michael, Dr., Im Grossen Garten 18, D-6054 Rodgau 2 (DE)**
Erfinder: **Weber, Georg, Wilhelm-Leuschner-Strasse 38, D-6106 Erzhausen (DE)**

**EP 0 144 013 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft neue Thienothiophenderivate der Formell

$$R^1-(A^1)_m-Z^1-A-(Z^2-A^2)_n-R^2 \quad \text{I}$$

worin

$R^1$ und $R^2$ jeweils H, eine Alkylgruppe mit 1–12 C-Atomen, worin auch eine oder zwei nicht benachbarte $CH_2$-Gruppen durch O-Atome, –CO– oder –CH=CH-Gruppen ersetzt sein können, F, Cl, Br, CN, –COOR oder –O–COR,

$A^1$ und $A^2$ jeweils unsubstituierte oder durch ein oder zwei F-, Cl-, Br-Atome und/oder CN-Gruppen und/oder $CH_3$-Gruppen substituierte 1,4-Phenylen-, 1,4-Cyclohexylen-, 1,3-Dioxan-2,5-diyl-, 1,3-Dithian-2,5-diyl, Tetrahydropyran-2,5-diyl, Pyridazin-3,6-diyl- oder das entsprechende N-oxid, Piperidin-1,4-diyl-, 1,4-Bicyclo(2,2,2)-octylen-, oder Pyrimidin-2,5-diylgruppen,

A eine unsubstituierte oder durch ein oder zwei Cl- und/oder Br-Atome substituierte Gruppe der Formel 1 oder 2

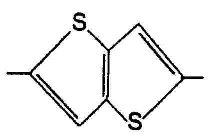

1

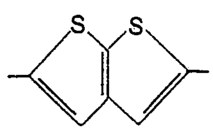

2

$Z^1$ und $Z^2$ jeweils –CO–O–, –O–CO–, $-CH_2CH_2-$, $-OCH_2-$, $-CH_2O-$ oder eine Einfachbindung,

R eine Alkylgruppe mit 1–10 C-Atomen

m 1 oder 2 und

n 0 oder 1

bedeuten,

wobei für m = 2 die beiden Gruppen $A^1$ gleich oder voneinander verschieden sein können.

Der Einfachheit halber bedeuten im folgenden Phe eine 1,4-Phenylengruppe, Cy eine 1,4-Cyclohexylengruppe, Dio eine 1,3-Dioxan-2,5-diylgruppe, Bi eine Bicyclo-(2,2,2)-octylengruppe, Pip eine Piperidin-1,4-diylgruppe, und Pyr eine Pyrimidin-2,5-diylgruppe, wobei diese Gruppen, insbesondere die 1,4-Phenylengruppe und/oder die 1,4-Cyclohexylengruppe unsubstituiert oder durch ein oder zwei F und/oder Cl- und/oder Br-Atome und/oder CN-Gruppen und/oder $CH_3$-Gruppen substituiert sein können.

Die Verbindungen der Formel I können wie ähnliche Verbindungen als Komponenten flüssigkristalliner Dielektrika verwendet werden, insbesondere für Displays, die auf dem Prinzip der verdrillten Zelle, den Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Phasen geeignet sind.

Es wurde gefunden, dass die Verbindungen der Formel I als Komponenten flüssigkristalliner Phasen vorzüglich geeignet sind. Insbesondere sind mit ihrer Hilfe stabile flüssigkristalline Phasen mit positiver oder negativer dielektrischer Anisotropie, breiten nematischen Bereichen, günstigen elastischen Eigenschaften und vergleichsweise niedriger Viskosität herstellbar.

Mit der Bereitstellung der Verbindungen der Formel I wird ausserdem ganz allgemeine die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie einer solchen Phase zu variieren. Die Verbindungen der Formel I eignen sich ferner als Zwischenprodukte zur Herstellung anderer Substanzen, die sich als Bestandteile flüssigkristalliner Phase verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, dass man eine Verbindung, die sonst der Formel I entspricht, aber an Stelle von H-Atomen eine oder mehrere reduzierbare Gruppen und/oder C–C-Bindungen enthält, mit einem reduzierenden Mittel behandelt,

oder dass man zur Herstellung von Estern der Formel I (worin $R^1$ und/oder $R^2$ $-O-COR_2$ oder –COOR bedeuten und/oder worin $Z^1$ und/oder $Z^2$ –CO–O– oder –O–CO– bedeuten) eine entsprechende Carbonsäure oder eines ihrer reaktionsfähigen Derivate mit einem entsprechenden Alkohol oder einem seiner reaktionsfähigen Derivate umsetzt,

oder dass man zur Herstellung von Dioxanderivaten der Formel I (worin $A^1$ und/oder $A^2$ 1,3-Dioxan-2,5-diyl bedeuten) einen entsprechenden Aldehyd mit einem entsprechenden Diol umsetzt,

oder dass man zur Herstellung von Nitrilen der Formel I (worin $R^1$ und/oder $R^2$ CN bedeuten) ein entsprechendes Carbonsäureamid dehydratisiert oder ein entsprechendes Carbonsäurehalogenid mit Sulfamid umsetzt,

oder dass man ein Thiophenderivat der Formel II,

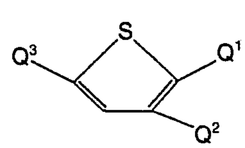

II

worin

einer der Reste $Q^1$ und $Q^2$ –CHO, der andere

$-SCH_2-Q^3$ und $Q^3$ $R^1-(A^1)_m-Z^1-$ oder $R^2-(A^2-Z^2)_n-$ bedeutet, kondensiert,

oder dass man zur Herstellung von Ethern der Formel I (worin $R^1$ und/oder $R^2$ eine Alkylgruppe bedeutet, worin eine oder zwei $CH_2$-Gruppen durch O-Atome ersetzt sind und/oder $Z^1$ und/oder $Z^2$ eine $-OCH_2$- oder $-CH_2O$-Gruppe ist) eine entsprechende Hydroxyverbindung verethert,

und/oder dass man gegebenenfalls eine Chlor- oder Bromverbindung der Formel I (worin $R^1$ und/oder $R^2$ Cl oder Br bedeuten) mit einem Cyanid umsetzt.

Weiterhin ist Gegenstand der Erfindung die Verwendung der Verbindungen der Formel I als Komponenten flüssigkristalliner Phasen.

Gegenstand der Erfindung sind ebenfalls flüssigkristalline Phasen, mit mindestens zwei flüssigkristallinen Komponenten, die zur Verbesserung der elastischen Eigenschaften mindestens eine flüssigkristalline Verbindung, enthaltend einen strukturellen Bestandteil der Formel 1 oder 2

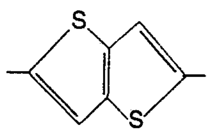

1

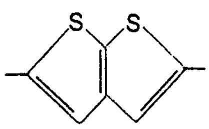

2

enthält.

Gegenstand der Erfindung sind ferner flüssigkristalline Phasen mit einem Gehalt an mindestens einer Verbindung der Formel I sowie Flüssigkristallanzeigeelemente, insbesondere elektrooptische Anzeigeelemente, die derartige Phasen enthalten.

Die Verbindungen der Formel I umfassen dementsprechend Verbindungen der Teilformeln Ia (mit zwei Ringen). Ib und Ic (mit jeweils drei Ringen) sowie Id (mit vier Ringen):

$R^1-A^1-Z^1-A-R^2$ Ia
$R^1-A^1-Z^1-A-A^2-R^2$ Ib
$R^1-(A^1)_2-Z^1-A-R^2$ Ic
$R^1-(A^1)_2-Z^1-A-Z^2-A^2-R^2$ Id

Die bevorzugten Verbindungen der Teilformel Ia umfassen solche der Teilformeln Ie bis Ij:

$R^1$-Phe-$Z^1$-A-$R^2$ Ie
$R^1$-Cy-$Z^1$-A-$R^2$ If
$R^1$-Dio-$Z^1$-A-$R^2$ Ig
$R^1$-Pip-$Z^1$-A-$R^2$ Ih
$R^1$-Bic-$Z^1$-A-$R^2$ Ii
$R^1$-Pyr-$Z^1$-A-$R^2$ Ij

Darunter sind diejenigen der Formeln Ie, If und Ig besonders bevorzugt.

Von den Verbindungen der Teilformeln Ib, Ic und Id sind diejenigen der Teilformeln Ik bis Iz besonders bevorzugt:

$R^1$-Phe-$Z^1$-A-$Z^2$-Phe-$R^2$ Ik
$R^1$-Dio-$Z^1$-A-$Z^2$-Cy-$R^2$ Il
$R^1$-Cy-$Z^1$-A-$Z^2$-Phe-$R^2$ Im
$R^1$-Cy-$Z^1$-A-$Z^2$-Cy-$R^2$ In
$R^1$-Phe-Phe-$Z^1$-A-$R^2$ Io
$R^1$-Phe-Cy-$Z^1$-A-$R^2$ Ip
$R^1$-Cy-Phe-$Z^1$-A-$R^2$ Iq
$R^1$-Cy-Cy-$Z^1$-A-$R^2$ Ir
$R^1$-Phe-Phe-$Z^1$-A-$Z^2$-Phe-$R^2$ Is
$R^1$-Phe-Phe-$Z^1$-A-$Z^2$-Cy-$R^2$ It
$R^1$-Phe-Cy-$Z^1$-A-$Z^2$-Phe-$R^2$ Iu
$R^1$-Phe-Cy-$Z^1$-A-$Z^2$-Cy-$R^2$ Iv
$R^1$-Cy-Phe-$Z^1$-A-$Z^2$-Phe-$R^2$ Iw
$R^1$-Cy-Phe-$Z^1$-A-$Z^2$-Cy-$R^2$ Ix
$R^1$-Cy-Cy-$Z^1$-A-$Z^2$-Phe-$R^2$ Iy
$R^1$-Cy-Cy-$Z^1$-A-$Z^2$-Cy-$R^2$ Iz

In den Verbindungen der vor- und nachstehenden Formeln bedeuten $R^1$ und $R^2$, die gleich oder verschieden sein können, vorzugsweise Alkyl, ferner Alkoxy (insbesondere, wenn diese Reste an einer Phe-Gruppe stehen) oder eine andere Oxaalkylgruppe.

Weiterhin bevorzugt sind Verbindungen der Formel I, worin einer der Reste $R^1$ und $R^2$ H, Halogen, insbesondere F, oder CN bedeuten.

$A^1$ und $A^2$ sind bevorzugt CY, Phe, Pyr, ferner bevorzugt Dio oder Pip; bevorzugt enthält die Verbindung der Formel I nicht mehr als einen der Reste Dio, Pip, Bic oder Pyr.

Weiterhin bevorzugt sind Verbindungen der Formel I, die mindestens einen cycloaliphatischen Ring (Cy, Dio, Bi oder Pip) enthalten.

A ist bevorzugt eine unsubstituierte Gruppe der Formel 1 oder 2.

$Z^1$ und $Z^2$, die gleich oder verschieden sein können, sind bevorzugt Einfachbindungen, -CO-O-, -O-CO- oder $CH_2CH_2$-Gruppen, in zweiter Linie bevorzugt -CO-O- oder -O-CO-Gruppen. Besonders bevorzugt sind erfindungsgemässe Verbindungen, worin eine der Gruppen $Z^1$ und $Z^2$ eine Einfachbindung und die andere eine -O-CO-, -CO-O- oder $-CH_2CH_2$-Gruppe ist. m ist vorzugsweise 1 oder 2, insbesondere 1, n ist vorzugsweise 0.

Falls $R^1$ und/oder $R^2$ Alkylreste bedeuten, in denen auch eine («Alkoxy» bzw. «Oxaalkyl») oder zwei («Alkoxy-alkoxy» bzw. «Dioxaalkyl») nicht benachbarte $CH_2$-Gruppen durch O-Atome ersetzt sein können, so können sie geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methyl, Octyl, Nonyl, Decyl, Undecyl, Dodecyl, Methoxy, Octoxy, Nonoxy, Decoxy, Undecoxy, Dodecoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Weiterhin bevorzugt sind Verbindungen der Formel I worin $R^2$ einen Alkylrest oder einen Alkoxyrest mit 3 bis 7 C-Atomen bedeutet. Ferner

bevorzugt sind Verbindungen der Formel I, worin einer der Reste $R^1$ und $R^2$ einen geradkettigen Alkylrest mit 2 bis 7 C-Atomen und der andere Rest eine geradkettige Alkoxygruppe mit 2 bis 12 C-Atomen bedeutet.

Verbindungen der Formeln I sowie Ia bis Iz mit verzweigten Flügelgruppen $R^1$ bzw. $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste $R^1$ und $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

In den Resten R sind die Alkylgruppen bzw. Alkoxygruppen ebenfalls vorzugsweise geradkettig und bedeuten insbesondere Methyl, Ethyl, Propyl, Butyl oder Pentyl, insbesondere Methyl, Propyl oder Pentyl.

Unter den Verbindungen der Formeln I sowie Ia bis Iz sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln Iaa bis Iat:

| | |
|---|---|
| $R^1$–$A^1$–$Z^1$–A–$R^2$ | Iaa |
| $R^1$–Phe–$Z^1$–A–$R^2$ | Iab |
| $R^1$–Phe–A–$R^2$ | Iac |
| $R^1$–Phe–CO–O–A–$R^2$ | Iad |
| $R^1$–Phe–O–CO–A–$R^2$ | Iae |
| $R^1$–Phe–$CH_2CH_2$–A–$R^2$ | Iaf |
| $R^1$–Phe–O–$CH_2$–A–$R^2$ | Iag |
| $R^1$–Phe–$CH_2$–O–A–$R^2$ | Iah |
| $R^1$–Cy–$Z^1$–A–$R^2$ | Iai |
| $R^1$–Cy–A–$R^2$ | Iaj |
| $R^1$–Cy–CO–O–A–$R^2$ | Iak |
| $R^1$–Cy–O–CO–A–$R^2$ | Ial |
| $R^1$–Cy–$CH_2CH_2$–A–$R^2$ | Iam |
| $R^1$–Cy–O–$CH_2$–A–$R^2$ | Ian |
| $R^1$–Cy–$CH_2$–O–A–$R^2$ | Iao |
| $R^1$–Dio–A–$R^2$ | Iap |
| $R^1$–Pip–A–$R^2$ | Iaq |
| $R^1$–Bic–A–$R^2$ | Iar |
| $R^1$–Pyr–A–$R^2$ | Ias |
| $R^1$–Phe–Phe–A–$R^2$ | Iat |

Unter den Verbindungen der Formel I, die eine 1,4-Cyclohexylen-, 1,3-Dioxan-2,5-diyl-, 1,3-Dithian-2,5-diyl-, Tetrahydropyran-2,5-diyl und/oder Piperidin-1,4-diyl-Gruppe enthalten, sind diejenigen bevorzugt, in denen diese Gruppen transsubstituiert sind.

Diejenigen der vorstehend genannten Formeln, die eine oder mehrere der Gruppen Dio, Pip und/ oder Pyr enthalten, umschliessen jeweils die beiden möglichen 2,5- bzw. 1,4-Stellungsisomeren. So umschliesst beispielsweise die Teilformel Iap die 2-$R^1$-5-(A-$R^2$)-1,3-dioxane und die 2-(A-$R^2$)-5-$R^1$-1,3-dioxane, die Teilformel Iaq die 1-$R^1$-4-(A-$R^2$)-piperidine und die 1-(A-$R^2$)-4-$R^1$-piperidine.

Falls Verbindungen der Formel I verzweigte Flügelgruppen $R_1$ und/oder $R_2$ aufweisen umfasst Formel I sowohl die Racemate als auch die optischen Antipoden sowie Gemische derselben.

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Bei der Synthese der Ausgangsstoffe können im Prinzip alle Methoden angewendet werden, die einerseits für die Verbindungen der Formel I bekannt sind, die an Stelle der Gruppe A andere Gruppen tragen, oder andererseits für die Thiophenderivate der Formel II bekannt sind, die an Stelle der Reste $Q^1$, $Q^1$ und/oder $Q^3$ andere Reste tragen. Der Fachmann kann die erforderlichen Synthesevarianten nach Routinemethoden ableiten.

Die Ausgangsstoffe können gewünschtenfalls auch in situ gebildet werden, derart, dass man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C–C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanrings einen Cyclohexenring oder Cyclohexanonring und/oder an Stelle einer $-CH_2CH_2$-Gruppe eine $-CH=CH$-Gruppe und/oder an Stelle einer $-CH_2$-Gruppe eine –CO-Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch Hydrierung entsprechender Sulfonate oder Halogenide mit Zink und einer Säure (z.B. Eisessig, Salzsäure) oder mit komplexen Hydriden (z.B. mit $NaBH_4$, zweckmässig in einem Lösungsmittel wie Dimethylsulfoxid oder mit $LiAlH_4$, zweckmässig in einem Lösungsmittel wie Diethylether, Dioxan, Tetrahydrofuran). Die Reduktion erfolgt vorzugsweise bei Temperaturen zwischen etwa 0° und etwa 100°.

Die Sulfonate oder Halogenide sind nach literaturbekannten Methoden beispielsweise aus den

entsprechenden Alkoholen erhältlich, die ihrerseits durch Reduktion der entsprechenden Ketone zugänglich sind. Verbindungen mit reduzierbaren C–C-Bindungen lassen sich nach literaturbekannten Methoden in Dihalogenide überführen, die dann nach den genannten Verfahren enthalogeniert werden können.

Ketone können auch nach den Methoden von Clemmensen (mit Zink, amalgamiertem Zink oder Zinn und Salzsäure, zweckmässig in wässrigalkoholischer Lösung oder in heterogener Phase mit Wasser/Toluol bei Temperaturen zwischen etwa 80 und 120°) oder Wolff-Kishner (mit Hydrazin, zweckmässig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder $-CH_2CH_2$-Brücken enthalten, reduziert werden.

Ester der Formel I ($R_1$ und/oder $R^2$ = –O–COR oder $Z^1$ und/oder $Z^2$ = –CO–O– oder –O–CO–) können auch durch Veresterung entsprechender Carbonsäuren der Formeln

R–COOH, $R^1$–$(A^1)_m$–COOH,
$R^1$–$(A^1)_m$–$Z^1$–A–COOH,
$R^2$–$(A^2)_n$–COOH oder $R^2$–$(A^2$–$Z^2)_n$–A–COOH

(oder ihrer reaktionsfähigen Derivate) mit Alkoholen bzw. Phenolen der Formeln

$R^1$–$(A^1)_m$–$Z^1$–A–$(Z^2$–$A^2)_n$–OH,
$R^2$–$(A^2$–$Z^2)_n$–A–$Z^1$–$(A^1)_m$–OH,
$R^2$–$(A^2$–$Z^2)_n$–A–OH, $R^2$–$(A^2)_n$–OH,
$R^1$–$(A^1)_m$–$Z^1$–A–OH oder $R^1$–$(A^1)_m$–OH

(oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride der Formeln

$R^1$–$(A^1)_m$–CO–O–$COCH_3$,
$R^1$–$(A^1)_m$–$Z^1$–A–CO–O–$COCH_3$,
$R^2$–$(A^2)_n$–CO–O–$COCH_3$ und
$R^2$–$(A^2$–$Z^2)_n$–A–CO–O–$COCH_3$, Azide oder Ester, insbesondere Alkylester mit 1–4 C-Atomen in der Alkylgruppe.

Als reaktionsfähige Derivate der genannten Alkohole bzw. Phenole kommen insbesondere die entsprechenden Metallalkoholate bzw. Phenolate der Formeln

$R^1$–$(A^1)_m$–$Z^1$–A–$(Z^2$–$A^2)_n$–OM,
$R^2$–$(A^2$–$Z^2)_n$–A–$Z^1$–$(A^1)_m$–OM, $R^2$–$(A^2$–$Z^2)_n$–A–OM,
$R^2$–$(A^2)_n$–OM, $R^1$–$(A^1)_m$–$Z^1$–A–OM und
$R^1$–$(A^1)_m$–OM

in Betracht, worin M ein Äquivalent eines Metalls, vorzugsweise eines Alkalimetalls wie Na oder K, bedeutet.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuss einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen −50° und +250°, vorzugsweise zwischen −20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat. Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, dass man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmässig bei Temperaturen zwischen etwa −25° und +20°.

Dioxanderivate der Formel I (worin eine der Gruppen $A^1$ und/oder $A^2$ eine 1,3-Dioxan-2,5-diyl-Gruppe bedeutet) werden zweckmässig durch Reaktion eines entsprechenden Aldehyds, z.B. der Formeln

$R^1$–$(A^1)_{m-1}$–CHO, $R^1$–$(A^1)_m$–$Z^1$–A–$Z^2$–CHO,
O=CH–$(A^1)_{m-1}$–$Z^1$–A–$(Z^2$–$A^2)_n$–$R^2$ bzw. O=CH–$R^2$

(oder eines seiner reaktionsfähigen Derivate) mit einem entsprechenden 1,3-Diol z.B. der Formeln

$(HOCH_2)_2CH$–$(A^1)_{m-1}$–$Z^1$–A–$(Z^2$–$A^2)_n$–$R^2$,
$(HOCH_2)_2CH$–$R^2$, $R^1$–$(A^1)_{m-1}$–$CH(CH_2OH_2)_2$ bzw.
$R^1$–$(A_1)_m$–$Z^1$–A–$Z^2CH(CH_2OH)_2$

(oder einem seiner reaktionsfähigen Derivate) hergestellt, vorzugsweise in Gegenwart eines inerten Lösungsmittels wie Benzol oder Toluol

und/oder eines Katalysators, z.B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangsstoffe eignen sich in erster Linie Acetale z.B. der Formeln
$R^1-(A^1)_{m-1}CH(OR^3)_2$, $R^1-(A^1)_m-Z^1-A-Z^2-CH(OR^3)_2$, $(R^3O)_2CH-(A^1)_{m-1}-Z^1-A-(Z^2-A^2)_n-R^2$, $(R^3O)_2-R^2$, $R^4-CH(OCH_2)_2CH-(A^1)_{m-1}-Z^1-A-(Z^2-A^2)_n-R^2$, $R^4-CH(OCH_2)_2CH-R^2$, $R^1-(A^1)_{m-1}-CH(CH_2O)_2CH-R^4$ bzw. $R^1-(A^1)_m-Z^1-A-Z^1CH(CH_2O)_2CHR^4$,
worin $R^3$ Alkyl mit 1–4 C-Atomen, zwei Reste $R^3$ zusammen auch Alkylen mit 2 oder 3 C-Atomen und $R^4$ H, Alkyl mit 1–4 C-Atomen oder Phenyl bedeuten.

Die genannten Aldehyde und 1,3-Diole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester erhältlich.

Zur Herstellung von Nitrilen der Formel I (worin $R^1$ und/oder $R^2$ CN bedeuten und/oder worin $A^1$ und/oder $A^2$ durch mindestens ein CN-Gruppe substituiert ist) können entsprechende Säureamide, z.B. solche, in denen an Stelle des Restes X eine $CONH_2$-Gruppe steht, dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride mit Sulfamid umsetzen, zweckmässig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Verbindungen der Formel I sind weiterhin erhältlich, in dem man ein Thiophenderivat der Formel II,

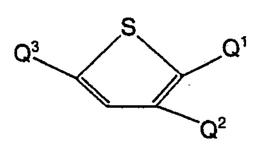

II

worin einer der Reste $Q^1$ und $Q^2$ –CHO, der andere $-SCH_2-Q^3$ und $Q^3$ $R^1-(A^1)_m-Z^1-$ oder $R^2-(A^2-Z^2)_n-$ bedeutet, kondensiert.

Die Herstellung der Verbindungen der Formel II und deren Kondensation kann nach an sich bekannten Methoden erfolgen, wie sie in der Literatur [z.B. V.P. Litvinov und Ya. L. Gold'farb, Izv. Akad. Nauk. SSSR, Ser. Khim., 2183 (1963)] beschrieben sind, und zwar unter Reaktionsbedingungen, die für die genannten Umsetzungen bekannt und geeignet sind. Dabei kann man auch von an sich bekannnten, hier nicht näher erwähnten Varianten Gebrauch machen.

Ether der Formel I (worin $R^1$ und/oder $R^2$ eine Alkylgruppe bedeutet, worin eine oder zwei $CH_2$-Gruppen durch O-Atome ersetzt sind, und/oder worin $Z^1$ und/oder $Z^2$ eine $-OCH_2$- oder $-CH_2O$-Gruppe ist) sind durch Veretherung entsprechender Hydroxyverbindungen, vorzugsweise entsprechender Phenole, erhältlich, wobei die Hydroxyverbindung zweckmässig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmässig in einem inerten Lösungsmittel wie Aceton, 1,2-Dimethoxyethan, DMF oder Dimethylsulfoxid oder auch einem Überschuss an wässriger oder wässrig-alkoholischer NaOH oder KOH bei Temperaturen zwischen etwa 20° und 100°.

Zur Herstellung von Nitrilen der Formel I (worin $R^1$ und/oder $R^2$ CN bedeuten und/oder worin $A^1$ und/oder $A^2$ durch mindestens eine CN-Gruppe substituiert ist) können auch entsprechende Chlor- oder Bromverbindungen der Formel I (worin $R^1$ und/oder $R^2$ Cl oder Br bedeuten und/oder worin $A^1$ und/oder $A^2$ durch mindestens ein Cl- oder Br-Atom substituiert ist) mit einem Cyanid umgesetzt werden, zweckmässig mit einem Metallcyanid wie NaCN, KCN oder $Cu_2(CN)_2$, z.B. in Gegenwart von Pyridin in einem inerten Lösungsmittel wie DMF oder N-Methylpyrrolidon bei Temperaturen zwischen 20° und 200°.

Die erfindungsgemässen flüssigkristallinen Phasen bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -Cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexylpyridazine, Phenyl- oder Cyclohexyldioxane, Phenyl- oder Cyclohexyldithiane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel VI charakterisieren,

$$R'–L–G–E–R'' \quad VI$$

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,4-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | –CH=CH– | –N(O)=N– |
| | –CH=CY– | –CH=N(O)– |
| | –C≡C– | $–CH_2–CH_2–$ |
| | –CO–O– | $–CH_2–O–$ |
| | –CO–S– | $–CH_2–S–$ |
| | –CH=N– | –COO–Phe–COO– |

oder eine C–C-Einfachbindung, Y Halogen, vorzugsweise Chlor, oder –CN, und R' und R'' Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden herstellbar.

Die erfindungsgemässen Phasen enthalten etwa 0,1 bis 99, vorzugsweise 10 bis 90%, einer oder mehrerer Verbindungen der Formel I. Weiterhin bevorzugt sind erfindungsgemässe Phasen, enthaltend 0,1 bis 70, insbesondere 0,5 bis 60%, einer oder mehrerer Verbindungen der Formel I.

Die Herstellung der erfindungsgemässen Phasen erfolgt in an sich üblicher Weise. In der Regel werden die Komponenten ineinander gelöst, zweckmässig bei erhöhter Temperatur.

Durch geeignete Zusätze können die flüssigkristallinen Phasen nach der Erfindung so modifiziert werden, dass sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol. Cryst. Liq. Cryst. Band 24, Seiten 249–258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 2 209 127, 2 240 864, 2 321 632, 2 338 281, 2 450 088, 2 637 430, 2 853 728 und 2 902 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben. «Übliche Aufarbeitung» bedeutet: man gibt Wasser hinzu, extrahiert mit Methylenchlorid, trennt ab, trocknet die organische Phase, dampft ein und reinigt das Produkt durch Kristallisation und/oder Chromatographie.

Beispiel 1

3,5 g
5-n-Pentyl-thieno[3,2-b]thiophen-2-carbonsäurechlorid
[erhältlich durch Umsetzung von
5-n-Pentyl-thieno[3,2-b]thiophen-5-carbonsäure
(F. 156°, K. 196°) mit Thionylchlorid]
in 20 ml Toluol tropft man zu einer Mischung aus 2,25 g 4-n-Pentylphenol, 1,08 g Pyridin und 30 ml Toluol, erhitzt 2 Stunden zum Sieden, lässt abkühlen, filtriert das Pyridinhydrochlorid ab und arbeitet wie üblich auf. Man erhält
5-n-Pentyl-thieno[3,2-b]thiophen-2-carbonsäure-4-n-pentylphenylester,
F. 58°, K. 93°.

Analog werden hergestellt:
5-Pentyl-thieno[3,2-b]thiophen-2-carbonsäure-4-ethylphenylester
5-Pentyl-thieno[3,2-b]thiophen-2-carbonsäure-4-propylphenylester
5-Pentyl-thieno[3,2-b]thiophen-2-carbonsäure-4-butylphenylester
5-Pentyl-thieno[3,2-b]thiophen-2-carbonsäure-4-heptylphenylester
5-Propyl-thieno[3,2-b]thiophen-2-carbonsäure-4-ethylphenylester
5-Propyl-thieno[3,2-b]thiophen-2-carbonsäure-4-propylphenylester
5-Propyl-thieno[3,2-b]thiophen-2-carbonsäure-4-butylphenylester
5-Propyl-thieno[3,2-b]thiophen-2-carbonsäure-4-pentylphenylester
5-Propyl-thieno[3,2-b]thiophen-2-carbonsäure-4-heptylphenylester
5-Butyl-thieno[3,2-b]thiophen-2-carbonsäure-4-ethylphenylester
5-Butyl-thieno[3,2-b]thiophen-2-carbonsäure-4-propylphenylester
5-Butyl-thieno[3,2-b]thiophen-2-carbonsäure-4-butylphenylester
5-Butyl-thieno[3,2-b]thiophen-2-carbonsäure-4-pentylphenylester
5-Butyl-thieno[3,2-b]thiophen-2-carbonsäure-4-heptylphenylester
5-Heptyl-thieno[3,2-b]thiophen-2-carbonsäure-4-ethylphenylester
5-Heptyl-thieno[3,2-b]thiophen-2-carbonsäure-4-propylphenylester
5-Heptyl-thieno[3,2-b]thiophen-2-carbonsäure-4-butylphenylester
5-Heptyl-thieno[3,2-b]thiophen-2-carbonsäure-4-pentylphenylester
5-Heptyl-thieno[3,2-b]thiophen-2-carbonsäure-4-heptylphenylester

5-Nonyl-thieno[3,2-b]thiophen-2-carbonsäure-4-ethylphenylester
5-Nonyl-thieno[3,2-b]thiophen-2-carbonsäure-4-propylphenylester
5-Nonyl-thieno[3,2-b]thiophen-2-carbonsäure-4-butylphenylester
5-Nonyl-thieno[3,2-b]thiophen-2-carbonsäure-4-pentylphenylester
5-Nonyl-thieno[3,2-b]thiophen-2-carbonsäure-4-heptylphenylester
5-Propyl-thieno[3,2-b]thiophen-2-carbonsäure-4-cyanphenylester
5-Butyl-thieno[3,2-b]thiophen-2-carbonsäure-4-cyanphenylester
5-Pentyl-thieno[3,2-b]thiophen-2-carbonsäure-4-cyanphenylester, F. 98°, K. 137°
5-Hexyl-thieno[3,2-b]thiophen-2-carbonsäure-4-cyanphenylester
5-Heptyl-thieno[3,2-b]thiophen-2-carbonsäure-4-cyanphenylester
5-Nonyl-thieno[3,2-b]thiophen-2-carbonsäure-4-cyanphenylester
5-Pentyl-thieno[3,2-b]thiophen-2-carbonsäure-4-fluorphenylester
5-Pentyl-thieno[3,2-b]thiophen-2-carbonsäure-4-chlorphenylester
5-Pentyl-thieno[3,2-b]thiophen-2-carbonsäure-4-bromphenylester
5-Pentyl-thieno[3,2-b]thiophen-2-carbonsäure-4-methoxyphenylester
5-Pentyl-thieno[3,2-b]thiophen-2-carbonsäure-4-ethoxyphenylester
5-Pentyl-thieno[3,2-b]thiophen-2-carbonsäure-4-propoxyphenylester
5-Pentyl-thieno[3,2,-b]thiophen-2-carbonsäure-4-butoxyphenylester
5-Pentyl-thieno[3,2-b]thiophen-2-carbonsäure-4-pentoxyphenylester
5-Pentyl-thieno[3,2-b]thiopen-2-carbonsäure-4-heptoxyphenylester
5-Pentyl-thieno[3,2-b]thiophen-2-carbonsäure-4-nonoxyphenylester
5-Pentyl-thieno[3,2-b]thiophen-2-carbonsäure-4′-propylbiphenylylester
5-Pentyl-thieno[3,2-b]thiophen-2-carbonsäure-4′-cyanbiphenylylester
5-Pentyl-thieno[3,2-b]thiophen-2-carbonsäure-2′-fluor-4′-ethylbiphenylylester
5-Pentyl-thieno[3,2-b]thiophen-2-carbonsäure-2-methyl-4′-propylbiphenylylester
5-Pentyl-thieno[3,2-b]thiophen-2-carbonsäure-4-(4-propylcyclohexyl)-phenylester
5-Pentyl-thieno[3,2-b]thiophen-2-carbonsäure-4-(1-cyan-4-propylcyclohexyl)-phenylester
5-Pentyl-thieno[3,2-b]thiophen-2-carbonsäure-4-(5-propyl-1,3-dioxan-2-yl)-phenylester
5-Pentyl-thieno[3,2-b]thiophen-2-carbonsäure-4′-(trans-4-ethylcyclohexylester)
5-Pentyl-thieno[3,2-b]thiophen-2-carbonsäure-4′-(trans-4-propylcyclohexylester), F. 45°, K. 93°.
5-Pentyl-thieno[3,2-b]thiophen-2-carbonsäure-4′-(trans-4-butylcyclohexylester)
5-Pentyl-thieno[3,2-b]thiophen-2-carbonsäure-4′-(trans-4-pentylcyclohexylester)
5-Pentyl-thieno[3,2-b]thiophen-2-carbonsäure-4′-(trans-4-heptylcyclohexylester)
5-Propyl-thieno[3,2-b]thiophen-2-carbonsäure-4′-(trans-4-ethylcyclohexylester)
5-Propyl-thieno[3,2-b]thiophen-2-carbonsäure-4′-(trans-4-propylcyclohexylester)
5-Propyl-thieno[3,2-b]thiophen-2-carbonsäure-4′-(trans-4-butylcyclohexylester)
5-Propyl-thieno[3,2-b]thiophen-2-carbonsäure-4′-(trans-4-pentylcyclohexylester)
5-Propyl-thieno[3,2-b]thiophen-2-carbonsäure-4′-(trans-4-heptylcyclohexylester)
5-Propyl-thieno[3,2-b]thiophen-2-carbonsäure-4′-(trans-4-ethoxycyclohexylester)
5-Propyl-thieno[3,2-b]thiophen-2-carbonsäure-4′-(trans-4-butoxycyclohexylester)
5-Propyl-thieno[3,2-b]thiophen-2-carbonsäure-4′-(trans,trans-4-ethylbicyclohex-4′-yl-ester)
5-Propyl-thieno[3,2-b]thiophen-2-carbonsäure-4′-(trans,trans-4-propylbicyclohex-4′-yl-ester)
5-Propyl-thieno[3,2-b]thiophen-2-carbonsäure-4′-(trans,trans-4-butylbicyclohex-4′-yl-ester)
5-Propyl-thieno[3,2-b]thiophen-2-carbonsäure-4′-(trans,trans-4-pentylbicyclohex-4′-yl-ester)
5-Propyl-thieno[3,2-b]thiophen-2-carbonsäure-4′-(trans,trans-4-heptylbicyclohex-4′-yl-ester)
5-Propyl-thieno[3,2-b]thiophen-2-carbonsäure-4′-(trans,trans-4-ethoxybicyclohex-4′-yl-ester)
5-Propyl-thieno[3,2-b]thiophen-2-carbonsäure-4′-(trans,trans,4-butoxybicyclohex-4′-yl-ester)
5-Propyl-thieno[3,2-b]tiophen-2-carbonsäure-4′-(trans,trans-4-propionyloxybicyclohex-4′-yl-ester)

Beispiel 2

5,75 g
2-[2-trans-5-n-Pentylcyclohexyl)-acetyl]-5-n-pentylthieno[3,2-b]thiophen
[erhältlich durch Umsetzung von
5-n-Pentylthieno[3,2-b]thiophen-2-carbonitril
(aus dem Säurechlorid durch Umsetzung mit Ammoniak und anschliessende Dehydratisierung des Amids mit Thionylchlorid] mit einer aus trans-4-Brommethyl-n-pentylcyclohexan hergestellten Grignardlösung] werden mit 2,5 g 85%igem Hydrazinhydrat, 3,2 g Kaliumhydroxid und 15 ml Triethylenglykol 2 Stunden gekocht, dann bis zum Erreichen einer Innentemperatur von 195° am absteigenden Kühler erhitzt und 4 Std. bei dieser Temperatur gehalten. Nach dem Abkühlen und Verdünnen mit Wasser wird nach üblicher Aufarbeitung 4,1 g
1-trans-4-Pentylcyclohexyl-2-[5-pentylthieno-[3,2-b]thien-2-yl]ethan
erhalten.

Analog werden hergestellt:

1-trans-4-Methylcyclohexyl-2-[5-pentylthieno-[3,2-b]thien-2-yl]ethan
1-trans-4-Ethylcyclohexyl-2-[5-pentylthieno-[3,2-b]thien-2-yl]ethan
1-trans-4-Propylcyclohexyl-2-[5-pentylthieno-[3,2-b]thien-2-yl]ethan
1-trans-4-Butylcyclohexyl-2-[5-pentylthieno-[3,2-b]thien-2-yl]ethan

1-trans-4-Heptylcyclohexyl-2-[5-pentylthieno-[3,2-b]thien-2-yl]ethan
1-trans-4-Nonylcyclohexyl-2-[5-pentylthieno-[3,2-b]thien-2-yl]ethan
1-trans-4-Methylcyclohexyl-2-[5-propylthieno-[3,2-b]thien-2-yl]ethan
1-trans-4-Ethylcyclohexyl-2-[5-propylthieno-[3,2-b]thien-2-yl]ethan
1-trans-4-Propylcyclohexyl-2-[5-propylthieno-[3,2-b]thien-2-yl]ethan
1-trans-4-Butylcyclohexyl-2-[5-propylthieno-[3,2-b]thien-2-yl]ethan
1-trans-4-Pentylcyclohexyl-2-[5-propylthieno-[3,2-b]thien-2-yl]ethan
1-trans-4-Heptylcyclohexyl-2-[5-propylthieno-[3,2-b]thien-2-yl]ethan
1-trans-4-Nonylcyclohexyl-2-[5-propylthieno-[3,2-b]thien-2-yl]ethan
1-trans-4-Methylcyclohexyl-2-[5-heptylthieno-[3,2-b]thien-2-yl]ethan
1-trans-4-Ethylcyclohexyl-2-[5-heptylthieno-[3,2-b]thien-2-yl]ethan
1-trans-4-Propylcyclohexyl-2-[5-heptylthieno-[3,2-b]thien-2-yl]ethan
1-trans-4-Butylcyclohexyl-2-[5-heptylthieno-[3,2-b]thien-2-yl]ethan
1-trans-4-Pentylcyclohexyl-2-[5-heptylthieno-[3,2-b]thien-2-yl]ethan
1-trans-4-Heptylcyclohexyl-2-[5-heptylthieno-[3,2-b]thien-2-yl]ethan
1-trans-4-Nonylcyclohexyl-2-[5-heptylthieno-[3,2-b]thien-2-yl]ethan
1-trans-4-Methylcyclohexyl-2-[5-(p-propylphenyl)-thieno[3,2-b]thien-2-yl]-ethan
1-trans-4-Ethylcyclohexyl-2-[5-(p-propylphenyl)-thieno[3,2-b]thien-2-yl]-ethan
1-trans-4-Propylcyclohexyl-2-[5-(p-propylphenyl)-thieno[3,2-b]thien-2-yl]-ethan
1-trans-4-Butylcyclohexyl-2-[5-(p-propylphenyl)-thieno[3,2-b]thien-2-yl]ethan
1-trans-4-Pentylcyclohexyl-2-[5-(p-propylphenyl)-thieno[3,2-b]thien-2-yl]-ethan
1-trans-4-heptylcyclohexyl-2-[5-[p-propylphenyl)-thieno[3,2-b]thien-2-yl]-ethan
1-trans-4-Methylcyclohexyl-2-[5-(p-cyanphenyl)-thieno[3,2-b]thien-2-yl]-ethan
1-trans-4-Ethylcyclohexyl-2-[5-(p-cyanphenyl)-thieno[3,2-b]thien-2-yl]-ethan
1-trans-4-Propylcyclohexyl-2-[5-(p-cyanphenyl)-thieno[3,2-b]thien-2-yl]-ethan
1-trans-4-Butylcyclohexyl-2-[5-(p-cyanphenyl)-thieno[3,2-b]thien-2-yl]-ethan
1-trans-4-Pentylcyclohexyl-2-[5-(p-cyanphenyl)-thieno[3,2-b]thien-2-yl]-ethan
1-trans-4-Heptylcyclohexyl-2-[5-(p-cyanphenyl)-thieno[3,2-b]thien-2-yl]-ethan
1-trans-4-Methylcyclohexyl-2-[5-(p-butoxyphenyl)-thieno[3,2-b]thien-2-yl]-ethan
1-trans-4-Ethylcyclohexyl-2-[5-(p-butoxyphenyl)-thieno[3,2-b]thien-2-yl]-ethan
1-trans-4-Propylcyclohexyl-2-[5-(p-butoxyphenyl)-thieno[3,2-b]thien-2-yl]-ethan
1-trans-4-Butylcyclohexyl-2-[5-(p-butoxyphenyl)-thieno[3,2-b]thien-2-yl]-ethan
1-trans-4-Pentylcyclohexyl-2-[5-(p-butoxyphenyl)-thieno[3,2-b]thien-2-yl]-ethan
1-trans-4-Heptylcyclohexyl-2-[5-(p-butoxyphenyl)-thieno[3,2-b]-thien-2-yl]-ethan
1-trans-4-Methylcyclohexyl-2-[5-(5-propylpyrimidin-2-yl)-thieno[3,2-b]thien-2-yl]ethan
1-trans-4-Ethylcyclohexyl-2-[5-(5-propylpyrimidin-2-yl)-thieno[3,2-b]thien-2-yl]-ethan
1-trans-4-Propylcyclohexyl-2-[5-(5-propylpyrimidin-2-yl)-thieno[3,2-b]thien-2-yl]-ethan
1-trans-4-Butylcyclohexyl-2-[5-(5-propylpyrimidin-2-yl)-thieno[3,2-b]thien-2-yl]-ethan
1-trans-4-Pentylcyclohexyl-2-[5-(5-propylpyrimidin-2-yl)-thieno[3,2-b]thien-2-yl]-ethan
1-trans-4-Heptylcyclohexyl-2-[5-(5-propylpyrimidin-2-yl)-thieno[3,2,-b]thien-2-yl]-ethan
1-p-Ethylphenyl-2-[5-pentyl-thieno[2,3-b]-thien-2-yl]-ethan
1-p-Butylphenyl-2-[5-pentyl-thieno[2,3-b]-thien-2-yl]-ethan
1-p-Pentylphenyl-2-[5-pentyl-thieno[2,3-b]-thien-2-yl]-ethan
1-p-Heptylphenyl-2-[5-pentyl-thieno[2,3-b]-thien-2-yl]-ethan
1-p-Cyanphenyl-2-[5-pentyl-thieno[2,3-b]-thien-2-yl]-ethan
1-p-Fluorphenyl-2-[5-pentyl-thieno[2,3-b]-thien-2-yl]-ethan
1-p-Chlorphenyl-2-[5-pentyl-thieno[2,3-b]-thien-2-yl]-ethan
1—4-Ethylcyclohexyl-2-[5-pentyl-thieno[2,3-b]-thien-2-yl]-ethan
1—4-Propylcyclohexyl-2-[5-pentyl-thieno[2,3-b]-thien-2-yl]-ethan
1—4-Butylcyclohexyl-2-[5-pentyl-thieno[2,3-b]-thien-2-yl]-ethan
1—4-Pentylcyclohexyl-2-[5-pentyl-thieno[2,3-b]-thien-2-yl]-ethan
1—4-Heptylcyclohexyl-2-[5-pentyl-thieno[2,3-b]-thien-2-yl]-ethan.

Beispiel 3

Zu einer aus 1,9 g Natrium und 60 ml Ethanol hergestellten Alkoholatlösung tropft man bei Raumtemperatur innerhalb von 5 min eine Lösung von 4,6 g
3-p-Cyanbenzylmercapto-2-formyl-5-(4-pentylcyclohexyl)-thiophen
in 25 ml Ethanol und kocht anschliessend 5 Stunden. Nach üblicher Aufarbeitung erhält man
2-p-Cyanphenyl-5-(4-pentylcyclohexyl)-thieno[3,2-b]thiophen.

Analog werden hergestellt:
2-p-Cyanphenyl-5-(4-propylcyclohexyl)-thieno-[3,2-b]thiophen
2-p-Cyanphenyl-5-(4-butylcyclohexyl)-thieno-[3,2-b]thiophen
2-p-Cyanphenyl-5-(4-heptylcyclohexyl)-thieno-[3,2-b]thiophen
2-p-Cyanphenyl-5-(4′-propylcyclohexylcyclohexyl)-thieno[3,2-b]thiophen
2-p-Cyanphenyl-5-(4′-butylcyclohexylcyclohexyl)-thieno[3,2-b]thiophen

2-p-Cyanphenyl-5-[4'-pentylcyclohexylcyclohexyl)-thieno[3,2-b]thiophen
2-p-Cyanphenyl-5-(4'-heptylcyclohexylcyclohexyl)-thieno[3,2-b]thiophen
2-p-Cyanphenyl-5-(p-4-propylcyclohexylphenyl)-thieno[3,2-b]thiophen
2-p-Cyanphenyl-5-(p-4-butylcyclohexylphenyl)-thieno[3,2-b]thiophen
2-p-Cyanphenyl-5-(p-4-pentylcyclohexylphenyl)-thieno[3,2-b]thiophen
2-p-Cyanphenyl-5-(p-4-heptylcyclohexylphenyl)-thieno[3,2-b]thiophen.

Beispiel 4

20,5 g 5-n-Heptyl-3-chlorthieno[3,2-b]thiophen-2-carbonsäurechlorid [erhältlich durch Umsetzung von 5-n-Heptylthiophen-2-acrylsäure (aus 5-n-Heptylthiophen-2-carbaldehyd und Malonsäure in Gegenwart von Pyridin/Piperidin) mit Thionylchlorid/Pyridin in Toluol] wird mit 13,6 g 4-n-Propylphenol und 8 g Pyridin in 100 ml Toluol 4 Stdn. auf 100° erhitzt. Nach üblicher Aufarbeitung erhält man 14,8 g 4-n-Propylphenyl-(5-n-heptyl-3-chlorthieno[3,2-b]-thiophen)-2-carboxylat.

Analog werden hergestellt:
4-Ethylphenyl-(5-n-heptyl-3-chlorthieno-[3,2-b]thiophen)-2-carboxylat
4-Butylphenyl-(5-n-heptyl-3-chlorthieno-[3,2-b]thiophen)-2-carboxylat
4-Pentylphenyl-(5-n-heptyl-3-chlorthieno-[3,2-b]thiophen)-2-carboxylat
4-Heptylphenyl-(5-n-heptyl-3-chlorthieno-[3,2-b]thiophen)-2-carboxylat
4-Nonylphenyl-(5-n-heptyl-3-chlorthieno-[3,2-b]thiophen)-2-carboxylat
4-Cyanphenyl-(5-n-heptyl-3-chlorthieno[3,2-b]-thiophen)-2-carboxylat
4-Fluorphenyl-(5-n-heptyl-3-chlorthieno-[3,2-b]thiophen)-2-carboxylat
4-Ethylcyclohexyl-(5-n-heptyl-3-chlorthieno-[3,2-b]thiophen)-2-carboxylat
4-Propylcyclohexyl-(5-n-heptyl-3-chlorthieno-[3,2-b]thiophen)-2-carboxylat
4-Butylcyclohexyl-(5-n-heptyl-3-chlorthieno-[3,2-b]thiophen)-2-carboxylat
4-Pentylcyclohexyl-(5-n-heptyl-3-chlorthieno-[3,2-b]thiophen)-2-carboxylat
4-Heptylcyclohexyl-(5-n-heptyl-3-chlorthieno-[3,2-b]thiophen)-2-carboxylat
4-Nonylcyclohexyl-(5-n-heptyl-3-chlorthieno-[3,2-b]thiophen)-2-carboxylat

Beispiel 5

20,4 g n-Octylthieno[2,3-b]thiophen-2-carbaldehyd (erhältlich aus 2-n-Octylthieno[2,3-b]thiophen durch Vilsmeyer-Formylierung mit Phosphoroxychlorid/Dimethylformamid) und 9,5 g 2-n-Pentylpropan-1,3-diol werden in 150 ml Toluol gelöst, mit einer Spatelspitze Toluolsulfonsäure versetzt und am Wasserabscheider erhitzt bis sich kein Wasser mehr abscheidet. Man wäscht mit Natriumhydrogencarbonatlösung die Säure aus, trocknet über Natriumsulfat und destilliert das Toluol ab. Das reine trans-2-(5n-Octylthieno[2,3-b]thien-2-yl)-5-n-pentyl-1,3-dioxan erhält man durch mehrmaliges Umkristallisieren aus Ethanol (17,5 g).

Analog werden hergestellt:
2-(5-n-Octylthieno[2,3-b]thien-2-yl)-5-ethyl-1,3-dioxan
2-(5-n-Octylthieno[2,3-b]thien-2-yl)-5-propyl-1,3-dioxan
2-(5-n-Octylthieno[2,3-b]thien-2-yl)-5-butyl-1,3-dioxan
2-(5-n-Octylthieno[2,3-b]thien-2-yl)-5-heptyl-1,3-dioxan
2-(5-n-Octylthieno[2,3-b]thien-2-yl)-5-nonyl-1,3-dioxan
2-(5-n-Pentylthieno[2,3-b]thien-2-yl)-5-ethyl-1,3-dioxan
2-(5-n-Pentylthieno[2,3-b]thien-2-yl)-5-propyl-1,3-dioxan
2-(5-n-Pentylthieno[2,3-b]thien-2-yl)-5-butyl-1,3-dioxan
2-(5-n-Pentylthieno[2,3-b]thien-2-yl)-5-pentyl-1,3-dioxan, F. 86°, K. 84,5° (monotrop)
2-(5-n-Pentylthieno[2,3-b]thien-2-yl)-5-heptyl-1,3-dioxan
2-(5-n-Pentylthieno[2,3-b]thien-2-yl)-5-nonyl-1,3-dioxan
2-(5-n-Propylthieno[2,3-b]thien-2-yl)-5-ethyl-1,3-dioxan
2-(5-n-Propylthieno[2,3-b]thien-2-yl)-5-propyl-1,3-dioxan
2-(5-n-Propylthieno[2,3-b]thien-2-yl)-5-butyl-1,3-dioxan
2-(5-n-Propylthieno[2,3-b]thien-2-yl)-5-pentyl-1,3-dioxan
2-(5-n-Propylthieno[2,3-b]thien-2-yl)-5-heptyl-1,3-dioxan
2-(5-n-Propylthieno[2,3-b]thien-2-yl)-5-nonyl-1,3-dioxan
2-(5-Cyanthieno[2,3-b)thien-2-yl)-5-ethyl-1,3-dioxan
2-(5-Cyanthieno[2,3-b]thien-2-yl)-5-propyl-1,3-dioxan
2-(5-Cyanthieno[2,3-b]thien-2-yl)-5-butyl-1,3-dioxan
2-(5-Cyanthieno[2,3-b]thien-2-yl)-5-pentyl-1,3-dioxan
2-(5-Cyanthieno[2,3-b]thien-2-yl)-5-heptyl-1,3-dioxan
2-(5-Cyanthieno[2,3-b]thien-2-yl)-5-nonyl-1,3-dioxan
2-(5-Cyanthieno[2,3-b]thien-2-yl)-5-(4-ethylcyclohexyl)-1,3-dioxan
2-(5-Cyanthieno[2,3-b]thien-2-yl)-5-(4-propylcyclohexyl)-1,3-dioxan
2-(5-Cyanthieno[2,3-b]thien-2-yl)-5-(4-butylcyclohexyl)-1,3-dioxan
2-(5-Cyanthieno[2,3-b]thien-2-yl)-5-(4-pentylcyclohexyl)-1,3-dioxan
2-(5-Cyanthieno[2,3-b]thien-2-yl)-5-(4-heptylcyclohexyl)-1,3-dioxan

2-(5-Cyanthieno[2,3-b]thien-2-yl)-5-(4-nonylcyclohexyl)-1,3-dioxan.

Beispiel 6

19 g

2-[4-(trans-4-n-Propylcyclohexyl)styryl]-5-n-pentylthiophen

(erhältlich durch Friedel-Crafts-Acylierung von 2-Pentylthiophen mit 4-(trans-4-n-Propylcyclohexyl)-phenylacetylchlorid, Reduktion des entstandenen Ketons mit Natriumboranat zum Alkohol und anschliessende Dehydratisierung) und 5 g Schwefel werden 3 Stunden auf 220° erhitzt. Anschliessende chromatographische Aufarbeitung ergibt 8,3 g

2-[4-trans-4-n-Propylcyclohexylphenyl]-5-n-pentylthieno[3,2-b]thiophen.

Analog werden hergestellt:

2-[4-(trans-4-Ethylcyclohexyl)-phenyl]-5-n-pentylthieno[3,2-b]thiophen
2-[4-(trans-4-Butylcyclohexyl)-phenyl]-5-n-pentylthieno[3,2-b]thiophen
2-[4-(trans-4-Pentylcyclohexyl)-phenyl]-5-n-pentylthieno[3,2-b]thiophen
2-[4-(trans-4-Heptylcyclohexyl)-phenyl]-5-n-pentylthieno[3,2-b]thiophen
2-[4-(trans-4-Nonylcyclohexyl)-phenyl]-5-n-pentylthieno[3,2-b]thiophen
2-[4-(trans-4-Ethylcyclohexyl)-phenyl]-5-n-heptylthieno[3,2-b]thiophen
2-[4-(trans-4-Butylcyclohexyl)-phenyl]-5-n-heptylthieno[3,2-b]thiophen
2-[4-(trans-4-Pentylcyclohexyl)-phenyl]-5-n-heptylthieno[3,2-b]thiophen
2-[4-(trans-4-Heptylcyclohexyl)-phenyl]-5-n-heptylthieno[3,2-b]thiophen
2-[4-(trans-4-Nonylcyclohexyl)-phenyl]-5-n-heptylthieno[3,2-b]thiophen
2-[4-(trans-4-Ethylcyclohexyl)-phenyl]-5-cyanthieno[3,2-b]thiophen
2-[4-(trans-4-Butylcyclohexyl)-phenyl]-5-cyanthieno[3,2-b]thiophen
2-[4-(trans-4-Pentylcyclohexyl)-phenyl]-5-cyanthieno[3,2-b]thiophen
2-[4-(trans-4-Heptylcyclohexyl)-phenyl]-5-cyanthieno[3,2-b]thiophen
2-[4-(trans-4-Nonylcyclohexyl)-phenyl]-5-cyanthieno[3,2-b]thiophen
2-[trans,trans-4′-Ethylcyclohexylcyclohexyl]-5-cyanthieno[3,2-b]thiophen
2-[trans,trans-4′-Butylcyclohexylcyclohexyl]-5-cyanthieno[3,2-b]thiophen
2-[trans,trans-4′-Pentylcyclohexylcyclohexyl]-5-cyanthieno[3,2-b]thiophen
2-[trans,trans-4′-Heptylcyclohexylcyclohexyl]-5-cyanthieno[3,2-b]thiophen
2-[trans,trans-4′-Nonylcyclohexylcyclohexyl]-5-cyanthieno[3,2-b]thiophen

2-(p-Ethylphenyl)-5-propylthieno[3,2-b]thiophen
2-(p-Propylphenyl)-5-propylthieno[3,2-b]thiophen
2-(p-Butylphenyl)-5-propylthieno[3,2-b]thiophen
2-(p-Pentylphenyl)-5-propylthieno[3,2-b]thiophen
2-(p-Heptylphenyl)-5-propylthieno[3,2-b]thiophen
2-(p-Cyanphenyl)-5-propylthieno[3,2-b]thiophen
2-(p-Methoxyphenyl)-5-propylthieno[3,2-b]thiophen
2-(p-Ethoxyphenyl)-5-propylthieno[3,2-b]thiophen
2-(p-Butoxyphenyl)-5-propylthieno[3,2-b]thiophen
2-(p-Pentoxyphenyl)-5-propylthieno[3,2-b]thiophen
2-(p-Heptoxyphenyl)-5-propylthieno[3,2-b]thiophen
2-(p-Nonoxyphenyl)-5-propylthieno[3,2-b]thiophen
2-(p-Decoxyphenyl)-5-propylthieno[3,2-b]thiophen
2-(p-Undecoxyphenyl)-5-propylthieno[3,2-b]thiophen
2-(p-Dodecoxyphenyl)-5-propylthieno[3,2-b]thiophen
2-(p-Ethylphenyl)-5-pentylthieno[3,2-b]thiophen
2-(p-Propylphenyl)-5-pentylthieno[3,2-b]thiophen
2-(p-Butylphenyl)-5-pentylthieno[3,2-b]thiophen
2-(p-Pentylphenyl)-5-pentylthieno[3,2-b]thiophen
2-(p-Heptylphenyl)-5-pentylthieno[3,2-b]thiophen
2-(p-Cyanphenyl)-5-pentylthieno[3,2-b]thiophen
2-(p-Methoxyphenyl)-5-pentylthieno[3,2-b]thiophen
2-(p-Ethoxyphenyl)-5-pentylthieno[3,2-b]thiophen
2-(p-Butoxyphenyl)-5-pentylthieno[3,2-b]thiophen
2-(p-Pentoxyphenyl)-5-pentylthieno[3,2-b]thiophen
2-(p-Heptoxyphenyl)-5-pentylthieno[3,2-b]thiophen
2-(p-Nonoxyphenyl)-5-pentylthieno[3,2-b]thiophen
2-(p-Decoxyphenyl)-5-pentylthieno[3,2-b]thiophen
2-(p-Undecoxyphenyl)-5-pentylthieno[3,2-b]thiophen
2-(p-Dodecoxyphenyl)-5-pentylthieno[3,2-b]thiophen

2-(p-Ethylphenyl)-5-hexylthieno[3,2-b]thiophen
2-(p-Propylphenyl)-5-hexylthieno[3,2-b]thiophen
2-(p-Butylphenyl)-5-hexylthieno[3,2-b]thiophen
2-(p-Pentylphenyl)-5-hexylthieno[3,2-b]thiophen
2-(p-Heptylphenyl)-5-hexylthieno[3,2-b]thiophen
2-(p-Cyanphenyl)-5-hexylthieno[3,2-b]thiophen
2-(p-Methoxyphenyl)-5-hexylthieno[3,2-b]thiophen

2-(p-Ethoxyphenyl)-5-hexylthieno[3,2-b]thiophen
2-(p-Butoxyphenyl)-5-hexylthieno[3,2-b]thiophen
3-(p-Pentoxyphenyl)-5-hexylthieno[3,2-b]thiophen
2-(p-Heptoxyphenyl)-5-hexylthieno[3,2-b]thiophen
2-(p-Nonoxyphenyl)-5-hexylthieno[3,2-b]thiophen
2-(p-Decoxyphenyl)-5-hexylthieno[3,2-b]thiophen
2-(p-Undecoxyphenyl)-5-hexylthieno[3,2-b]thiophen
2-(p-Dodecoxyphenyl)-5-hexylthieno[3,2-b]thiophen

2-(4'-Ethylbiphenyl-4-yl)-5-propylthieno[3,2-b]-thiophen
2-(4'-Propylbiphenyl-4-yl)-5-propylthieno[3,2-b]-thiophen
2-(4'-Butylbiphenyl-4-yl)-5-propylthieno[3,2-b]-thiophen
2-(4'-Pentylbiphenyl-4-yl)-5-propylthieno[3,2-b]-thiophen
2-(4'-Heptylbiphenyl-4-yl)-5-propylthieno[3,2-b]-thiophen
2-(4'-Cyanobiphenyl-4-yl)-5-propylthieno[3,2-b]-thiophen
2-(4'-Methoxybiphenyl-4-yl)-5-propylthieno[3,2-b]-thiophen
2-(4'-Ethoxybiphenyl-4-yl)-5-propylthieno[3,2-b]-thiophen
2-(4'-Butoxybiphenyl-4-yl)-5-propylthieno[3,2-b]-thiophen
2-(4'-Pentoxybiphenyl-4-yl)-5-propylthieno[3,2-b]-thiophen
2-(4'-Heptoxybiphenyl-4-yl)-5-propylthieno[3,2-b]-thiophen
2-(4'-Nonoxybiphenyl-4-yl)-5-propylthieno[3,2-b]-thiophen
2-(4'-Decoxybiphenyl-4-yl)-5-propylthieno[3,2-b]-thiophen
2-(4'-Undecoxybiphenyl-4-yl)-5-propylthieno-[3,2-b]thiophen
2-(4'-Dodecoxybiphenyl-4-yl)-5-propylthieno-[3,2-b]thiophen
2-(4'-Ethylbiphenyl-4-yl)-5-pentylthieno[3,2-b]-thiophen
2-(4'-Propylbiphenyl-4-yl)-5-pentylthieno[3,2-b]-thiophen
2-(4'-Butylbiphenyl-4-yl)-5-pentylthieno[3,2-b]-thiophen
2-(4'-Pentylbiphenyl-4-yl)-5-pentylthieno[3,2-b]-thiophen
2-(4'-Heptylbiphenyl-4-yl)-5-pentylthieno[3,2-b]-thiophen
2-(4'-Cyanbiphenyl-4-yl)-5-pentylthieno[3,2-b]-thiophen
2-(4'-Methoxybiphenyl-4-yl)-5-pentylthieno[3,2-b]-thiophen
2-(4'-Ethoxybiphenyl-4-yl)-5-pentylthieno[3,2-b]-thiophen
2-(4'-Butoxybiphenyl-4-yl)-5-pentylthieno[3,2-b]-thiophen
2-(4'-Pentoxybiphenyl-4-yl)-5-pentylthieno[3,2-b]-thiophen
2-(4'-Heptoxybiphenyl-4-yl)-5-pentylthieno[3,2-b]-thiophen
2-(4'-Nonoxybiphenyl-4-yl)-5-pentylthieno[3,2-b]-thiophen
2-(4'-Decoxybiphenyl-4-yl)-5-pentylthieno[3,2-b]-thiophen
2-(4'-Undecoxybiphenyl-4-yl)-5-pentylthieno-[3,2-b]thiophen
2-(4'-Dodecoxybiphenyl-4-yl)-5-pentylthieno-[3,2-b]thiophen

Beispiel 7

Ein Gemisch aus 12 g 5-n-Pentylthieno[3,2-b]thiophen-2-carbamidin (erhältlich aus dem Nitril durch Umsetzung mit Ethanol/Salzsäure und anschliessend mit Ammoniak) und 11,4 g n-Pentylmalondialdehydtetraethylacetal wird unter Rühren 12 Stunden auf 160° (Badtemperatur) erhitzt. Die nach dem Abkühlen erhaltenen Kristalle werden chromatografisch und durch Kristallisation gereinigt. Man erhält 2-(5-n-Pentylthieno[3,2-b]thiophen-2-yl)-5-n-pentylpyrimidin, F. 118°, K. 106° (monotrop).

Analog werden hergestellt:

2-(5-Pentylthieno[3,2-b]thiophen-2-yl)-5-propylpyrimidin
2-(5-Pentylthieno[3,2-b]thiophen-2-yl)-5-butylpyrimidin
2-(5-Pentylthieno[3,2-b]thiophen-2-yl)-5-hexylpyrimidin
2-(5-Pentylthieno[3,2-b]thiophen-2-yl)-5-heptylpyrimidin, F. 102°, K. 106°
2-(5-Pentylthieno[3,2-b]thiophen-2-yl)-5-nonylpyrimidin
2-(5-Propylthieno[3,2-b]thiophen-2-yl)-5-ethylpyrimidin
2-(5-Propylthieno[3,2-b]thiophen-2-yl)-5-propylpyrimidin
2-(5-Propylthieno[3,2-b]thiophen-2-yl)-5-butylpyrimidin.
2-(5-Propylthieno[3,2-b]thiophen-2-yl)-5-pentylpyrimidin
2-(5-Propylthieno[3,2-b]thiophen-2-yl)-5-hexylpyrimidin
2-(5-Propylthieno[3,2-b]thiophen-2-yl)-5-heptylpyrimidin
2-(5-Heptylthieno[3,2-b]thiophen-2-yl)-5-ethylpyrimidin
2-(5-Heptylthieno[3,2-b]thiophen-2-yl)-5-propylpyrimidin
2-(5-Heptylthieno[3,2-b]thiophen-2-yl)-5-butylpyrimidin
2-(5-Heptylthieno[3,2-b]thiophen-2-yl)-5-pentylpyrimidin
2-(5-Heptylthieno[3,2-b]thiophen-2-yl)-5-hexylpyrimidin
2-(5-Heptylthieno[3,2-b]thiophen-2-yl)-5-heptylpyrimidin
2-(5-Cyanthieno[3,2-b]thiophen-2-yl)-5-ethylpyrimidin
2-(5-Cyanthieno[3,2-b]thiophen-2-yl)-5-propylpyrimidin
2-(5-Cyanthieno[3,2-b]thiophen-2-yl)-5-butylpyrimidin
2-(5-Cyanthieno[3,2-b]thiophen-2-yl)-5-pentylpyrimidin
2-(5-Cyanthieno[3,2-b]thiophen-2-yl)-5-hexylpyrimidin
2-(5-Cyanthieno[3,2-b]thiophen-2-yl)-5-heptylpyrimidin
2-(5-Methoxythieno[3,2-b]thiophen-2-yl)-5-ethylpyrimidin
2-(5-Methoxythieno[3,2-b]thiophen-2-yl)-5-propylpyrimidin
2-(5-Methoxythieno[3,2-b]thiophen-2-yl)-5-butylpyrimidin
2-(5-Methoxythieno[3,2-b]thiophen-2-yl)-5-pentylpyrimidin

2-(5-Methoxythieno[3,2-b]thiophen-2-yl)-5-hexylpyrimidin
2-(5-Methoxythieno[3,2-b]thiophen-2-yl)-5-heptylpyrimidin
2-(5-Ethoxythieno[3,2-b]thiophen-2-yl)-5-ethylpyrimidin
2-(5-Ethoxythieno[3,2-b]thiophen-2-yl)-5-propylpyrimidin
2-(5-Ethoxythieno[3,2-b]thiophen-2-yl)-5-butylpyrimidin
2-(5-Ethoxythieno[3,2-b]thiophen-2-yl)-5-pentylpyrimidin
2-(5-Ethoxythieno[3,2-b]thiophen-2-yl)-5-hexylpyrimidin
2-(5-Ethoxythieno[3,2-b]thiophen-2-yl)-5-heptylpyrimidin
2-(5-Butoxythieno[3,2-b]thiophen-2-yl)-5-ethylpyrimidin
2-(5-Butoxythieno[3,2-b]thiophen-2-yl)-5-propylpyrimidin
2-(5-Butoxythieno[3,2-b]thiophen-2-yl)-5-butylpyrimidin
2-(5-Butoxythieno[3,2-b]thiophen-2-yl)-5-pentylpyrimidin
2-(5-Butoxythieno[3,2-b]thiophen-2-yl)-5-hexylpyrimidin
2-(5-Butoxythieno[3,2-b]thiophen-2-yl)-5-heptylpyrimidin
2-(5-Hexoxythieno[3,2-b]thiophen-2-yl)-5-ethylpyrimidin
2-(5-Hexoxythieno[3,2-b]thiophen-2-yl)-5-propylpyrimidin
2-(5-Hexoxythieno[3,2-b]thiophen-2-yl)-5-butylpyrimidin
2-(5-Hexoxythieno[3,2-b]thiophen-2-yl)-5-pentylpyrimidin
2-(5-Hexoxythieno[3,2-b]thiophen-2-yl)-5-hexylpyrimidin
2-(5-Hexoxythieno[3,2-b]thiophen-2-yl)-5-heptylpyrimidin
2-(5-Octoxythieno[3,2-b]thiophen-2-yl)-5-ethylpyrimidin
2-(5-Octoxythieno[3,2-b]thiophen-2-yl)-5-propylpyrimidin
2-(5-Octoxythieno[3,2-b]thiophen-2-yl)-5-butylpyrimidin
2-(5-Octoxythieno[3,2-b]thiophen-2-yl)-5-pentylpyrimidin
2-(5-Octoxythieno[3,2-b]thiophen-2-yl)-5-hexylpyrimidin
2-(5-Octoxythieno[3,2-b]thiophen-2-yl)-5-heptylpyrimidin
2-(5-Nonoxythieno[3,2-b]thiophen-2-yl)-5-ethylpyrimidin
2-(5-Nonoxythieno[3,2-b]thiophen-2-yl)-5-propylpyrimidin
2-(5-Nonoxythieno[3,2-b]thiophen-2-yl)-5-butylpyrimidin
2-(5-Nonoxythieno[3,2-b]thiophen-2-yl)-5-pentylpyrimidin
2-(5-Nonoxythieno[3,2-b]thiophen-2-yl)-5-hexylpyrimidin
2-(5-Nonoxythieno[3,2-b]thiophen-2-yl)-5-heptylpyrimidin
2-(5-Decoxythieno[3,2-b]thiophen-2-yl)-5-ethylpyrimidin
2-(5-Decoxythieno[3,2-b]thiophen-2-yl)-5-propylpyrimidin
2-(5-Decoxythieno[3,2-b]thiophen-2-yl)-5-butylpyrimidin
2-(5-Decoxythieno[3,2-b]thiophen-2-yl)-5-pentylpyrimidin
2-(5-Decoxythieno[3,2-b]thiophen-2-yl)-5-hexylpyrimidin
2-(5-Decoxythieno[3,2-b]thiophen-2-yl)-5-heptylpyrimidin
2-(5-Undecoxythieno[3,2-b]thiophen-2-yl)-5-ethylpyrimidin
2-(5-Undecoxythieno[3,2-b]thiophen-2-yl)-5-propylpyrimidin
2-(5-Undecoxythieno[3,2-b]thiophen-2-yl)-5-butylpyrimidin
2-(5-Undecoxythieno[3,2-b]thiophen-2-yl)-5-pentylpyrimidin
2-(5-Undecoxythieno[3,2-b]thiophen-2-yl)-5-hexylpyrimidin
2-(5-Undecoxythieno[3,2-b]thiophen-2-yl)-5-heptylpyrimidin
2-(5-Dodecoxythieno[3,2-b]thiophen-2-yl)-5-ethylpyrimidin
2-(5-Dodecoxythieno[3,2-b]thiophen-2-yl)-5-propylpyrimidin
2-(5-Dodecoxythieno[3,2-b]thiophen-2-yl)-5-butylpyrimidin
2-(5-Dodecoxythieno[3,2-b]thiophen-2-yl)-5-pentylpyrimidin
2-(5-Dodecoxythieno[3,2-b]thiophen-2-yl)-5-hexylpyrimidin
2-(5-Dodecoxythieno[3,2-b]thiophen-2-yl)-5-heptylpyrimidin

Beispiel 8

Ein Gemisch aus 3,5 g 5-n-Pentyl-thieno[2,3-b]thiophen-2-carbonsäurechlorid [erhältlich durch Umsetzung von 5-n-Pentyl-thieno[2,3-b]thiophen-2-carbonsäure (F. 188°, K. 187° (monotrop)) mit Thionylchlorid; die Säure ist durch Umsetzung von 5-n-Pentyl-3-formyl-2-thienylmercapto)-essigsäureethylester [erhältlich durch Umsetzung von 2-n-Pentylthiophen mit Butyllithium, Schwefel und Chloressigsäureethylester und anschliessende Vilsmeyer-Formylierung des erhaltenen 5-n-Pentyl-2-thienylmercapto-essigesters] mit Natriumethylat/Ethanol erhältlich] in 20 ml Toluol tropft man zu einer Mischung aus 2,25 g 4-n-Pentylphenol, 1,08 g Pyridin und 30 ml Toluol, erhitzt 2 Stunden zum Sieden, lässt abkühlen, filtriert das Pyridinhydrochlorid ab und arbeitet wie üblich auf. Man erhält

5-n-Pentyl-thieno[2,3-b]thiophen-2-carbonsäure-4-n-pentylphenylester, F. 51,9°, K. 90,5°.

Analog werden hergestellt:
5-Pentyl-thieno[2,3-b]thiophen-2-carbonsäure-4-ethylphenylester

5-Pentyl-thieno[2,3-b]thiophen-2-carbonsäure-4-propylphenylester
5-Pentyl-thieno[2,3-b]thiophen-2-carbonsäure-4-butylphenylester
5-Pentyl-thieno[2,3-b]thiophen-2-carbonsäure-4-heptylphenylester
5-Propyl-thieno[2,3-b]thiophen-2-carbonsäure-4-ethylphenylester
5-Propyl-thieno[2,3-b]thiophen-2-carbonsäure-4-propylphenylester
5-Propyl-thieno[2,3-b]thiophen-2-carbonsäure-4-butylphenylester
5-Propyl-thieno[2,3-b]thiophen-2-carbonsäure-4-pentylphenylester
5-Propyl-thieno[2,3-b]thiophen-2-carbonsäure-4-heptylphenylester
5-Butyl-thieno[2,3-b]thiophen-2-carbonsäure-4-ethylphenylester
5-Butyl-thieno[2,3-b]thiophen-2-carbonsäure-4-propylphenylester
5-Butyl-thieno[2,3-b]thiophen-2-carbonsäure-4-butylphenylester
5-Butyl-thieno[2,3-b]thiophen-2-carbonsäure-4-pentylphenylester
5-Butyl-thieno[2,3-b]thiophen-2-carbonsäure-4-heptylphenylester
5-Heptyl-thieno[2,3-b]thiophen-2-carbonsäure-4-ethylphenylester
5-Heptyl-thieno[2,3-b]thiophen-2-carbonsäure-4-propylphenylester
5-Heptyl-thieno[2,3-b]thiophen-2-carbonsäure-4-butylphenylester
5-Heptyl-thieno[2,3-b]thiophen-2-carbonsäure-4-pentylphenylester
5-Heptyl-thieno[2,3-b]thiophen-2-carbonsäure-4-heptylphenylester
5-Nonyl-thieno[2,3-b]thiophen-2-carbonsäure-4-ethylphenylester
5-Nonyl-thieno[2,3-b]thiophen-2-carbonsäure-4-propylphenylester
5-Nonyl-thieno[2,3-b]thiophen-2-carbonsäure-4-butylphenylester
5-Nonyl-thieno[2,3-b]thiophen-2-carbonsäure-4-pentylphenylester
5-Nonyl-thieno[2,3-b]thiophen-2-carbonsäure-4-heptylphenylester
5-Propyl-thieno[2,3-b]thiophen-2-carbonsäure-4-cyanphenylester
5-Butyl-thieno[2,3-b]thiophen-2-carbonsäure-4-cyanphenylester
5-Pentyl-thieno[2,3-b]thiophen-2-carbonsäure-4-cyanphenylester, F. 85°, K. 129,2°
5-Hexyl-thieno[2,3-b]thiophen-2-carbonsäure-4-cyanphenylester
5-Heptyl-thieno[2,3-b]thiophen-2-carbonsäure-4-cyanphenylester
5-Nonyl-thieno[2,3-b]thiophen-2-carbonsäure-4-cyanphenylester
5-Pentyl-thieno[2,3-b]thiophen-2-carbonsäure-4-fluorphenylester
5-Pentyl-thieno[2,3-b]thiophen-2-carbonsäure-4-chlorphenylester
5-Pentyl-thieno[2,3-b]thiophen-2-carbonsäure-4-bromphenylester
5-Pentyl-thieno[2,3-b]thiophen-2-carbonsäure-4-methoxyphenylester
5-Pentyl-thieno[2,3-b]thiophen-2-carbonsäure-4-ethoxyphenylester
5-Pentyl-thieno[2,3-b]thiophen-2-carbonsäure-4-propoxyphenylester
5-Pentyl-thieno[2,3-b]thiophen-2-carbonsäure-4-butoxyphenylester
5-Pentyl-thieno[2,3-b]thiophen-2-carbonsäure-4-pentoxyphenylester
5-Pentyl-thieno[2,3-b]thiophen-2-carbonsäure-4-heptoxyphenylester
5-Pentyl-thieno[2,3-b]thiophen-2-carbonsäure-4-nonoxyphenylester
5-Pentyl-thieno[2,3-b]thiophen-2-carbonsäure-4′-propylbiphenylester
5-Pentyl-thieno[2,3-b]thiophen-2-carbonsäure-4′-cyanbiphenylester
5-Pentyl-thieno[2,3-b]thiophen-2-carbonsäure-2′-fluor-4′-ethylbiphenylester
5-Pentyl-thieno[2,3-b]thiophen-2-carbonsäure-2-methyl-4′-propylbiphenylester
5-Pentyl-thieno[2,3-b]thiophen-2-carbonsäure-4-(4-propylcyclohexyl)-phenylester
5-Pentyl-thieno[2,3-b]thiophen-2-carbonsäure-4-(1-cyan-4-propylcyclohexyl)-phenylester
5-Pentyl-thieno[2,3-b]thiophen-2-carbonsäure-4-(5-propyl-1,3-dioxan-2-yl)-phenylester.
5-Pentyl-thieno[2,3-b]thiophen-2-carbonsäure-4′-(trans-4-ethylcyclohexylester
5-Pentyl-thieno[2,3-b]thiophen-2-carbonsäure-4′-(trans-4-propylcyclohexylester), F. 60°, K. 91°
5-Pentyl-thieno[2,3-b]thiophen-2-carbonsäure-4′-(trans-4-butylcyclohexylester
5-Pentyl-thieno[2,3-b]thiophen-2-carbonsäure-4′-(trans-4-pentylcyclohexylester
5-Pentyl-thieno[2,3-b]thiophen-2-carbonsäure-4′-(trans-4-heptylcyclohexylester
5-Propyl-thieno[2,3-b]thiophen-2-carbonsäure-4′-(trans-4-ethylcyclohexylester)
5-Propyl-thieno[2,3-b]thiophen-2-carbonsäure-4′-(trans-4-propylcyclohexylester)
5-Propyl-thieno[2,3-b]thiophen-2-carbonsäure-4′-(trans-4-butylcyclohexylester)
5-Propyl-thieno[2,3-b]thiophen-2-carbonsäure-4′-(trans-4-pentylcyclohexylester)
5-Propyl-thieno[2,3-b]thiophen-2-carbonsäure-4′-(trans-4-heptylcyclohexylester)
5-Propyl-thieno[2,3-b]thiophen-2-carbonsäure-4′-(trans-4-ethoxycyclohexylester)
5-Propyl-thieno[2,3-b]thiophen-2-carbonsäure-4′-(trans-4-butoxycyclohexylester)
5-Propyl-thieno[2,3-b]thiophen-2-carbonsäure-4′-(trans,trans-4-ethylbicyclohex-4′-yl-ester)
5-Propyl-thieno[2,3-b]thiophen-2-carbonsäure-4′-(trans,trans-4-propylbicyclohex-4′-yl-ester)
5-Propyl-thieno[2,3-b]thiophen-2-carbonsäure-4′-(trans,trans-4-butylbicyclohex-4′-yl-ester)
5-Propyl-thieno[2,3-b]thiophen-2-carbonsäure-4′-(trans,trans-4-pentylbicyclohex-4′-y-ester)

5-Propyl-thieno[2,3-b]thiophen-2-carbonsäure-4′-(trans,trans-4-heptylbicyclohex-4′-yl-ester)

Es folgen Beispiele für erfindungsgemässe flüssigkristalline Phasen mit einem Gehalt an mindestens einer Verbindung der Formel I:

Beispiel A

Eine flüssigkristalline Phase aus
15% p-trans-4-Ethylcyclohexylbenzonitril,
15% p-trans-4-Butylcyclohexylbenzonitril,
10% 4-Ethyl-4′-cyanbiphenyl,
13% 4-Butyl-4′-cyanbiphenyl,
11% 4-Cyan-4′-(trans-4-pentylcyclohexyl)-biphenyl,
6% 4-p-Cyanphenyl-4′-pentylbiphenyl,
15% 5-Pentylthieno[3,2-b]thiophen-2-carbonsäure-4-pentylphenylester und
15% 5-Propylthieno[3,2-b]thiophen-2-carbonsäure-4-propylphenylester
hat einen Schmelzpunkt von −8°, einen Klärpunkt von 67° und eine Viskosität von 29 cSt.

Beispiel B

Eine flüssigkristalline Phase aus
8% 2-p-Cyanphenyl-5-ethyl-1,3-dioxan,
9% 2-p-Cyanphenyl-5-propyl-1,3-dioxan,
4% p-Ethylbenzoesäure-(p-cyanphenylester,
3% p-Propylbenzoesäure-(p-cyanphenylester),
11% p-trans-4-Ethylcyclohexylbenzonitril,
13% p-trans-4-Butylcyclohexylbenzonitril,
8% 4-Ethyl-4′-cyanphenyl,
12% 4-Butyl-4′-cyanbiphenyl,
5% p-trans-4-Pentylcyclohexyl-benzoesäure-(p-cyanphenylester),
9% 5-Propylthieno[3,2-b]thiophen-2-carbonsäure-4-cyanphenylester,
9% 5-Pentylthieno[3,2-b]thiophen-2-carbonsäure-4-cyanphenylester und
9% 5-Heptylthieno[3,2-b]thiophen-2-carbonsäure-4-cyanphenylester
hat einen Schmelzpunkt von −11°, einen Klärpunkt von 61° und eine Viskosität von 36 cSt. Ein elektrooptisches Anzeigeelement mit dieser Phase als Dielektrikum weist eine niedrige Schwellenspannung von $V_{10}$ = 1,15 V auf.

Beispiel C

Eine flüssigkristalline Phase aus
10% 2-p-Cyanphenyl-5-ethyl-1,3-dioxan,
10% 2-p-Cyanphenyl-5-propyl-1,3-dioxan,
16% 2-p-Cyanphenyl-5-butyl-1,3-dioxan,
10% 2-p-Cyanphenyl-5-pentyl-1,3-dioxan,
18% 5-Propylthieno[3,2-b]thiophen-2-carbonsäure-4-propylphenylester,
18% 5-Pentylthieno[3,2-b]thiophen-2-carbonsäure-4-pentylphenylester und
18% 5-Pentylthieno[3,2-b]thiophen-2-carbonsäure-4-heptylphenylester
hat einen Schmelzpunkt von −10°, einen Klärpunkt von 62° und eine Viskosität von 31 cSt.

Beispiel D

Eine flüssigkristalline Phase aus
4% 2-p-Cyanphenyl-5-propyl-1,3-dioxan,
5% 2-p-Cyanphenyl-5-butyl-1,3-dioxan,
4% 2-p-Cyanphenyl-5-pentyl-1,3-dioxan,
10% trans-4-Propylcyclohexancarbonsäure-(p-methoxyphenylester),
11% trans-4-Propylcyclohexancarbonsäure-(p-ethoxyphenylester),
15% trans-4-Butylcyclohexancarbonsäure-(p-methoxyphenylester),
10% trans-4-Butylcyclohexancarbonsäure-(p-ethoxyphenylester)
10% trans-4-Pentylcyclohexancarbonsäure-(p-methoxyphenylester),
13% trans-4-Pentylcyclohexancarbonsäure-(p-pentylphenylester),
6% 5-Propylthieno[3,2-b]thiophen-2-carbonsäure-4-cyanphenylester,
7% 5-Pentylthieno[3,2-b]thiophen-2-carbonsäure-4-cyanphenylester und
5% 5-Heptylthieno[3,2-b]thiophen-2-carbonsäure-4-cyanphenylester
hat einen Schmelzpunkt von −12°, einen Klärpunkt von 71° und eine Viskosität von 18 cSt. In einem elektrooptischen Anzeigeelement zeigt dieses Dielektrikum gute Multiplexeigenschaften (1:32).

Beispiel E

Eine flüssigkristalline Phase aus
18,0% p-trans-4-Propylcyclohexylbenzonitril,
13,5% p-trans-4-Butylcyclohexylbenzonitril,
24,5% p-trans-4-Pentylcyclohexylbenzonitril,
15,5% p-trans-4-Heptylcyclohexylbenzonitril,
7,0% 4-Cyan-4′-(trans-4-pentylcyclohexyl)-biphenyl,
7,0% 4-(trans-4-Pentylcyclohexyl)-4′-(trans-4-propylcyclohexyl)-biphenyl,
6,0% 4-p-Cyanphenyl-4′-pentyl-biphenyl,
8,0% trans-4-Butylcyclohexancarbonsäure-(p-trans-propylcyclohexylphenylester) und
0,5% 5-Pentylthieno[3,2-b]thiophen-2-carbonsäure-4-cyanphenylester
hat einen Schmelzpunkt von −14° und einen Klärpunkt von 88°.

Beispiel F

Eine flüssigkristalline Phase aus
14% p-trans-4-Propylcyclohexylbenzonitril,
16% 2-p-Cyanphenyl-5-butyl-1,3-dioxan,
10% 2-p-Cyanphenyl-5-pentyl-1,3-dioxan,
14% 5-Propylthieno[3,2-b]thiophen-2-carbonsäure-4-cyanphenylester,
10% 5-Pentylthieno[3,2-b]thiophen-2-carbonsäure-4-cyanphenylester,
8% 5-Heptylthieno[3,2-b]thiophen-2-carbonsäure-4-cyanphenylester,
16% 5-Propyl-thieno[3,2-b]thiophen-2-carbonsäure-4-propylphenylester und
12% 5-Pentyl-thieno[3,2-b]thiophen-2-carbonsäure-4-pentylphenylester
ist ein gutes Host-Material für pleochroitische Farbstoffe.

Beispiel G

Eine flüssigkristalline Phase aus
66,6% p-Methoxybenzoesäure-(p-pentylphenylester),

33,3% p-Hexoxybenzoesäure-(p-pentylphenyl-ester) und
0,1% 5-Propylthieno[3,2-b]thiophen-2-carbonsäure-4-propylphenylester
hat einen Schmelzpunkt von 13°.

**Patentansprüche**

1. Thienothiophenderivate der Formel I

$$R^1-(A^1)_m-Z^1-A-(Z^2-A^2)_n-R^2 \qquad I$$

worin

$R^1$ und $R^2$ jeweils H, eine Alkylgruppe mit 1–12 C-Atomen, worin auch eine oder zwei nicht benachbarte $CH_2$-Gruppen durch O-Atome, –CO- oder –CH=CH-Gruppen ersetzt sein können, F, Cl, Br, CN, –COOR oder –O–COR,

$A^1$ und $A^2$ jeweils unsubstituierte oder durch ein oder zwei F-, Cl-, Br-Atome und/oder CN-Gruppen und/oder $CH_3$-Gruppen substituierte 1,4-Phenylen-, 1,4-Cyclohexylen-, 1,3-Dioxan-2,5-diyl-, 1,3-Dithian-2,5-diyl, Tetrahydropyran-2,5-diyl, Pyridazin-3,6-diyl – oder das entsprechende N-oxid, Piperidin-1,4-diyl-, 1,4-Bicyclo(2,2,2)-octylen-, oder Pyrimidin-2,5-diylgruppen,

A eine unsubstituierte oder durch ein oder zwei Cl- und/oder Br-Atome substituierte Gruppe der Formel 1 oder 2

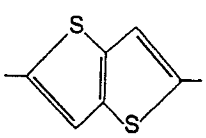

1

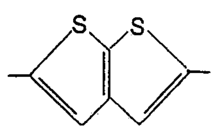

2

$Z^1$ und $Z^2$ jeweils –CO–O–, –O–CO–, $–CH_2CH–$, $–OCH_2–$, $–CH_2O–$ oder eine Einfachbindung,

R eine Akylgruppe mit 1–10 C-Atomen

m 1 oder 2 und

n 0 oder 1

bedeuten,

wobei für m = 2 die beiden Gruppen $A^1$ gleich oder voneinander verschieden sein können.

2. Verfahren zur Herstellung von Thienothiophenderivaten der Formel I nach Anspruch 1, dadurch gekennzeichnet, dass man eine Verbindung, die sonst der Formel I entspricht, aber an Stelle von H-Atomen eine oder mehrere reduzierbare Gruppen und/oder C–C-Bindungen enthält, mit einem reduzierenden Mittel behandelt, oder dass man zur Herstellung von Estern der Formel I (worin $R^1$ und/oder $R^2$ –O–COR oder –COOR bedeuten und/oder worin $Z^1$ und/oder $Z^2$ –CO–O– oder –O–CO– bedeuten) eine entsprechende Carbonsäure oder eines ihrer reaktionsfähigen Derivate mit einem entsprechenden Alkohol oder einem seiner reaktionsfähigen Derivate umsetzt,

oder dass man zur Herstellung von Dioxanderivaten der Formel I (worin $A^1$ und/oder $A^2$ 1,3-Dioxan-2,5-diyl bedeuten) einen entsprechenden Aldehyd mit einem entsprechenden Diol umsetzt,

oder dass man zur Herstellung von Nitrilen der Formel I (worin $R^1$ und/oder $R^2$ CN bedeuten) ein entsprechendes Carbonsäureamid dehydratisiert oder ein entsprechendes Carbonsäurehalogenid mit Sulfamid umsetzt,

oder dass man ein Thiophenderivat der Formel II,

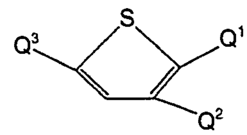

II

worin

einer der Reste $Q^1$ und $Q^2$ –CHO, der andere $–SCH_2–Q^3$ und $Q^3$ $R^1–(A^1)_m–Z^1–$ oder $R^2–(A^2–Z^2)_n–$ bedeutet, kondensiert,

oder dass man zur Herstellung von Ethern der Formel I (worin $R^1$ und/oder $R^2$ eine Alkylgruppe bedeutet, worin eine oder zwei $CH_2$-Gruppen durch O-Atome ersetzt sind und/oder $Z^1$ und/oder $Z^2$ eine $–OCH_2$- oder $–CO_2O$-Gruppe ist) eine entsprechende Hydroxyverbindung verethert,

und/oder dass man gegebenenfalls eine Chlor- oder Bromverbindung der Formel I (worin $R^1$ und/oder $R^2$ Cl oder Br bedeuten) mit einem Cyanid umsetzt.

3. Verwendung der Verbindungen der Formel I nach Anspruch 1 als Komponenten flüssigkristalliner Phasen.

4. Flüssigkristalline Phase, dadurch gekennzeichnet, dass sie mindestens eine Verbindung der Formel I enthält.

5. Flüssigkristalline Phase mit mindestens zwei flüssigkristallinen Komponenten, dadurch gekennzeichnet, dass sie zur Verbesserung der elastischen Eigenschaften mindestens eine flüssigkristalline Verbindung, enthaltend einen strukturellen Bestandteil der Formel 1 oder 2

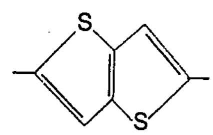

1

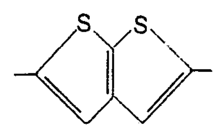

2

enthält.

6. Flüssigkristall-Anzeigeelement, dadurch gekennzeichnet, dass es eine flüssigkristalline Phase nach Anspruch 4 oder 5 enthält.

7. Elektrooptisches Anzeigeelement, dadurch gekennzeichnet, dass es als flüssigkristallines Dielektrikum eine flüssigkristalline Phase nach Anspruch 4 oder 5 enthält.

**Claims**

1. A thienothiophene derivative of the formula I

$$R^1-(A^1)_m-Z^1-A-(Z^2-A^2)_n-R^2 \qquad I$$

in which

$R^1$ and $R^2$ are each H or an alkyl group having 1–12 C-atoms, in which, furthermore, one or two non-adjacent $CH_2$ groups can be replaced by O

atoms, -CO- or $-CH=CH$-groups, or are each F, Cl, Br, CN, -COOR or -O-COR,

$A^1$ and $A^2$ are each 1,4-phenylene, 1,4-cyclohexylene, 1,3-dioxane-2,5-diyl, 1,3-dithiane-2,5-diyl, tetrahydropyran-2,5-diyl, pyridazine-3,6-diyl or the corresponding N-oxide, piperidine-1,4-diyl, 1,4-bicyclo[2.2.2]octylene or pyrimidine-2,5-diyl groups which are unsubstituted by one or two F, Cl or Br atoms and/or CN groups and/or $CH_3$ groups,

A is a group of the formula 1 or 2

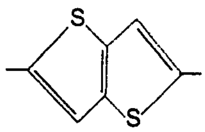
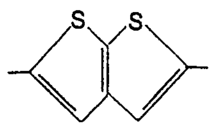

1 2

which is unsubstituted or substituted by one or two Cl and/or Br atoms,

$Z^1$ and $Z^2$ are each -CO-O-, -O-CO-, $-CH_2CH_2-$, $-OCH_2-$, $CH_2O-$ or a single bond,

R is an alkyl group having 1–10 C atoms,

m is 1 or 2 and

n is 0 or 1, and where m is 2, the two groups $A^1$ can be identical or different.

2. A process for the preparation of thienothiophene derivatives of the formula I according to Claim 1, characterised in that a compound which contains one or more reducible groups and/or C-C bonds in place of H atoms, but otherwise corresponds to the formula I, is treated with a reducing agent, or, for the preparation of esters of the formula I (in which $R^1$ and/or $R^2$ are -O-COR or -COOR, and/or in which $Z^1$ and/or $Z^2$ are -CO-O- or -O-CO-), an appropriate carboxylic acid, or one of its reactive derivatives, is reacted with an appropriate alcohol or with one of its reactive derivatives,

or, for the preparation of dioxane derivatives of the formula I (in which $A^1$ and/oder $A^2$ are 1,3-dioxane-2,5-diyl), an appropriate aldehyde is reacted with an appropriate diol,

or, for the preparation of nitriles of the formula I (in which $R^1$ and/or $R^2$ are CN), an appropriate carboximide is dehydrated or an appropriate carboxylic acid halide is reacted with sulfamide,

or a thiophene derivative of the formula II

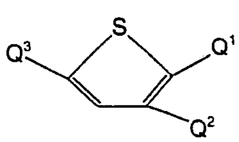

II

in which one of the radicals $Q^1$ and $Q^2$ is -CHO, and the other is $-SCH_2-Q$, and $Q^3$ is $R^1-(A^1)_m-Z^1-$ or $R^2-(A^2-Z^2)_n-$, is condensed,

or, for the preparation of ethers of the formula I (in which $R^1$ and $R^2$ are each alkyl groups, or $R^1$ or $R^2$ is an alkyl group, in which one or two $CH_2$ groups are replaced by O atoms, and/or $Z^1$ and $Z^2$ are each $-OCH_2$- or $-CH_2O$-groups or $Z^1$ or $Z^2$ is a $-OCH_2$- or $-CH_2O$-group), an appropriate hydroxy compound is etherified, and/or, if desired, a chlorine or bromine compound of the formula I (in which $R^1$ and/or $R^2$ are Cl or Br), is reacted with a cyanide.

3. Use of the compounds of the formula I according to Claim 1 as components of liquid crystalline phases.

4. A liquid crystalline phase, characterised in that it contains at least one compound of the formula I.

5. A liquid crystalline phase comprising at least two liquid-crystal components, characterised in that it contains at least one compound having a structural element of the formula 1 or 2

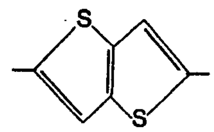
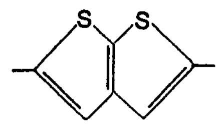

1 2

for improving the elastic properties.

6. A liquid crystal display element, characterised in that it contains a liquid crystalline phase according to Claim 4 or 5.

7. An electro-optical display element, characterised in that it contains as a liquid crystalline dielectric, a liquid crystalline phase according to Claim 4 or 5.

**Revendications**

1. Dérivés du thiénothiophène répondant à la formule I

$$R^1-(A^1)_m-Z^1-A-(Z^2-A^2)_n-R^2 \qquad I$$

dans laquelle

$R^1$ et $R^2$ représentent chacun H, un groupe alkyle en C1–C2 dans lequel 1 ou 2 groupes $CH_2$ non voisins peuvent également être remplacés par des atomes d'O, des groupes -CO- ou $-CO=CH-$, F, Cl, Br, CN, -COOR ou -O-COR,

$A^1$ et $A^2$ représentent chacun un groupe 1,4-phénylène, 1,4-cyclohexylène, 1,3-dioxanne-2,5-diyle, 1,3-dithianne-2,5-diyle, tétrahydropyranne-2,5-diyle, pyridazine-3,6-diyle non substitué ou substitué par 1 ou 2 atomes de F, de Cl, de Br et/ou groupes CN et/ou $CH_3$, ou le N-oxyde correspondant, des groupes pipéridine-1,4-diyle, 1,4-bicyclo-(2,2,2)-octylène ou pyrimidine-2,5-diyle,

A représente un groupe de formule 1 ou 2

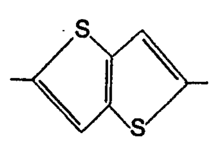
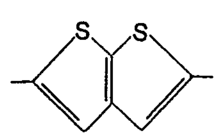

1 2

non substitué ou substitué par 1 ou 2 atomes de Cl et/ou de Br,

$Z^1$ et $Z^2$ représentent chacun -CO-O-, -O-CO-, $-CH_2CH_2-$, $-OCH_2-$, $-CH_2O-$ ou une liaison simple,

R représente un groupe alkyle en C1–C10

m est égal à 1 ou 2 et
n est égal à 0 ou 1,
les deux groupes $A^1$ pouvant être identiques ou différents lorsque m = 2.

2. Procédé de préparation des dérivés du thiénothiophène de formule I selon la revendication 1, caractérisé en ce que l'on traite par un agent réducteur un composé répondant par ailleurs à la formule I mais contenant, à la place d'atomes d'hydrogène, un ou plusieurs groupes réductibles et/ou liaisons C–C,

ou bien, pour la préparation des esters de formule I (dans lesquels $R^1$ et/ou $R^2$ représentent –O–COR ou –COOR et/ou dans lesquels $Z^1$ et/ou $Z^2$ représentent –CO–O– ou –O–CO–), on fait réagir un acide carboxylique correspondant ou l'un de ses dérivés réactifs avec un alcool correspondant ou l'un de ses dérivés réactifs,

ou bien, pour la préparation des dérivés du dioxanne de formule I (dans lesquels $A^1$ et/ou $A^2$ représentent des groupes 1,3-dioxanne-2,5-diyle), on fait réagir un aldéhyde correspondant avec un diol correspondant,

ou bien, pour la préparation des nitriles de formule I (dans lesquels $R^1$ et/ou $R^2$ représentent CN), on déshydrate un carboxamide correspondant ou bien on fait réagir un halogénure d'acide carboxylique correspondant avec le sulfonamide,

ou bien on condense un dérivé du thiophène répondant à la formule II,

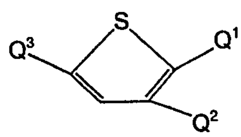

II

dans laquelle
l'un des symboles $Q^1$ et $Q^2$ représente –CHO et l'autre $-SCH_2-Q^3$ et $Q^3$ représente $R^1-(A^1)_m-Z^1-$ ou $R^2-(A^2-Z^2)_n-$,

ou bien, pour la préparation des éthers de formule I (dans lesquels $R^1$ et/ou $R^2$ représentent un groupe alkyle dans lequel 1 ou 2 groupes $CH_2$ peuvent être remplacés par des atomes d'O et/ou $Z^1$ et $Z^2$ représentent des groupes $-OCH_2-$ ou $-CH_2O-$, on éthérifie un composé hydroxylé correspondant, et/ou on fait réagir le cas échéant un dérivé chloré ou bromé de formule I (dans lequel $R^1$ et/ou $R^2$ représentent Cl ou Br) avec un cyanure.

3. Utilisation des composés de formule I selon la revendication 1 en tant que composant de phases à cristaux liquides.

4. Phase à cristaux liquides caractérisée en ce qu'elle contient au moins un composé de formule I.

5. Phase à cristaux liquides à au moins deux composants à cristaux liquides, caractérisé en ce que, pour l'amélioration des propriétés d'élasticité, elle contient au moins un composé à cristaux liquides contenant un constituant de structure de formule 1 ou 2

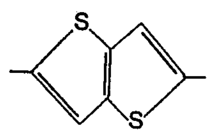

1

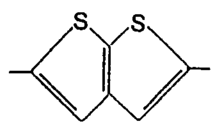

2

6. Elément d'affichage à cristaux liquides caractérisé en ce qu'il contient une phase à cristaux liquides selon la revendication 4 ou 5.

7. Elément d'affichage électro-optique caractérisé en ce qu'il contient en tant que diélectrique à cristaux liquides une phase à cristaux liquides selon la revendication 4 ou 5.